# EUROPEAN PATENT SPECIFICATION

(11) **EP 0 561 850 B1**
(45) Date of publication and mention of the grant of the patent: **12.07.2000**
(21) Application number: 91920890.0
(22) Date of filing: 05.12.1991
(51) Int. Cl.: C07D 401/14, C07D 471/04, C07D 498/06, C07D 521/00, A61K 31/47

(54) **7-SUBSTITUTED-6-FLUORO-1,4-DIHYDRO-4-OXO-QUINOLINE-3-CARBOXYLIC ACID COMPOUNDS USEFUL AS ANTIBACTERIAL AGENTS**
7-SUBSTITUIERTE-6-FLUOR-1,4-DIHYDRO-4-OXO-CHINOLIN-3-CARBONSÄUREDERIVATE ALS ANTIBAKTERIELLE WIRKSTOFFE
COMPOSES DE 6-FLUORO-1,4-DIHYDRO-4-OXO-QUINOLINE-3-ACIDE CARBOXYLIQUE SUBSTITUE EN POSITION 7, UTILISES COMME AGENTS ANTIBACTERIENS

(30) Priority: 05.12.1990 US 621716
(43) Date of publication of application: 29.09.1993
(73) Proprietor: NAEJA Pharmaceutical Inc., Edmonton Alberta T6E 5V2 (CA)
(72) Inventor: SINGH, Rajeshwar, Edmonton, Alberta T6K 1Z2 (CA); SINGH, Inder Pal, Edmonton, Alberta T6L 6L9 (CA); THOMAS, George, Edmonton, Alberta T6H 5G1 (CA); SINGH, Maya Prakash, Edmonton, Alberta T6L 2W1 (CA); MICETICH, Ronald George, Sherwood Park, Alberta T6E 5V2 (CA); FAHTI-AFSHAR, Rakhshandeh, Edmonton, Alberta T6J 5J2 (CA); DOERKSEN, Thomas, Roger, Edmonton, Alberta T6E 2L9 (CA)
(74) Representative: Pidgeon, Robert John
(86) International application number: CA9100435
(87) International publication number: WO9210492

(56) References cited:
- EP-A- 0 172 651
- EP-A- 0 203 488
- EP-A- 0 350 733
- EP-A- 0 387 802
- EP-A- 0 388 298
- Chemical Abstracts, volume 104, 1986, (Columbus, Ohio, US) see page 712, abstract 109495g, & JP, A, 60166681 (FUJISAWA PHARMACEUTICAL CO. LTD) 29 August 1985, see abstract compound with RN=100501-49-3
- Abstracts of the 30th Interscience Conference on Antimicrobial Agents and Chemotherapy, Atlanta, October 21-24, 1990, Abstract No. 395

## Description

### Background of the Invention

Many clinically important antibacterial agents, collectively known as fluoroquinolones, have been discovered. Quinolones which possess a substituted 1,4-dihydro-4-oxo-quinoline-3-carboxylic acid moiety and which have the general structural formula given below are described in J. Med. Chem. 23,1358 (1980.). 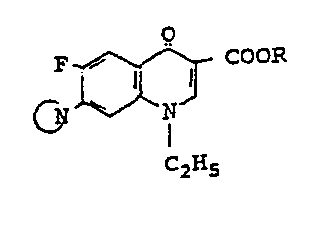 wherein N) may be piperazinyl or the like. Belgian Patent 899399 discloses 1-cyclopropyl-7-piperazinyldihydroquinoline carboxylic acid compounds of the formula 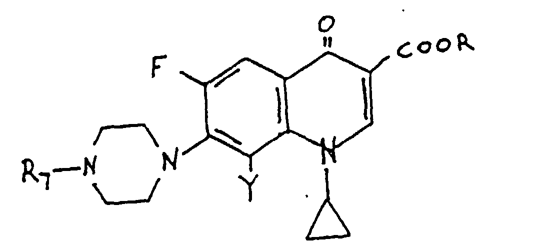 wherein R₇ is H or CH₃ and Y is Cl, F or CH₃. Japanese Patent No. 174367/1983, South African Patent No. 8502369, European Patent Nos. 172651 and 221463, 119087, U.S. Patent No. 4556658, 1985 and J. Med. Chem. 1990, 33 (1645-1656), disclose compounds represented by the following general formula having an amino group at position 5. 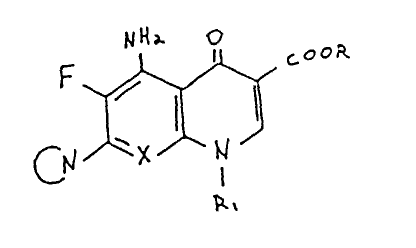 wherein R₁ is ethyl, cyclopropyl; X is CH, CF, C-CH₃; and N) is piperazinyl or the like.

J. Med. Chem. 1988, 31 (1598-1611) discloses compounds of the general formula 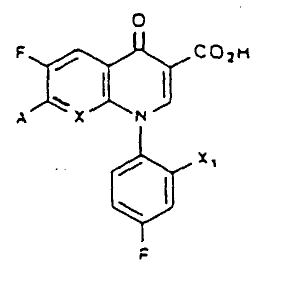 The reference discloses a compound according to this general formula in which A is 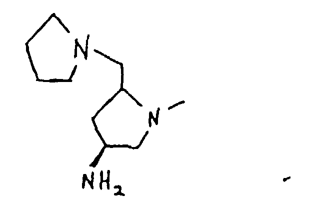 X is CH and X₁ is F.

J. Med. Chem. 1990, 33 (849-854) discloses compounds of the general formula 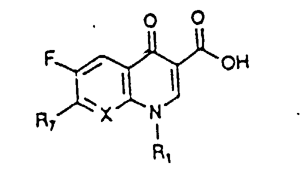 The reference discloses a compound according to this general formula in which X is N, R₁ is cyclopropyl and R₇ is

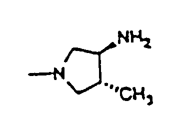

European Patent No. 347,851 discloses quinoline compounds which have NH₂ or F at the 5-position.

European Patent No. 362,759 discloses compounds having the general formula 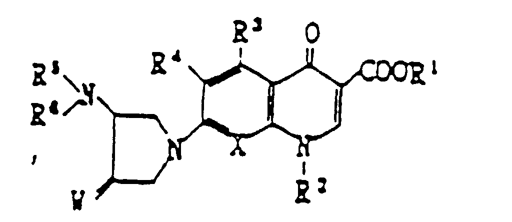 in which W is C₁-C₃ alkylidene.

Abstracts of the 30th Interscience Conference on Antimicrobial Agents and Chemotherapy, Atlanta, October 21-24, 1990, Abstract No. 395 discloses compounds having the formula 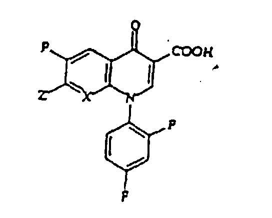 in which X is CH or N and in which Z is 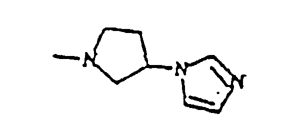 Abstracts of the 30th Interscience Conference on Antimicrobial Agents and Chemotherapy also discloses compounds having the formula 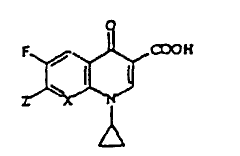 in which X is CH or N and Z is

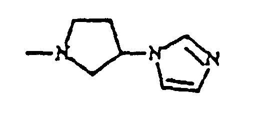

Some of the above disclosed compounds are clinically useful. However, there exists a continuing need to develop new antibacterial agents because the effectiveness of existing antibacterial agents diminishes as strains of pathogens develop resistance. In addition, certain antibiotics exhibit unsuitable pharmaceutical properties and exert serious adverse side effects in humans.

### Summary of the Invention

The present invention is based on the discovery that certain 7-substituted-6-fluoro-1,4-dihydro-4-oxo-quinoline-3-carboxylic acid compounds exhibit excellent activity against sensitive and resistant Gram-positive and moderate activity against Gram-negative bacteria.

In accordance with the present invention there is provided 7-substituted-6-fluoro-1,4-dihydro-4-oxo-quinoline-3-carboxylic acid compounds of the formula: 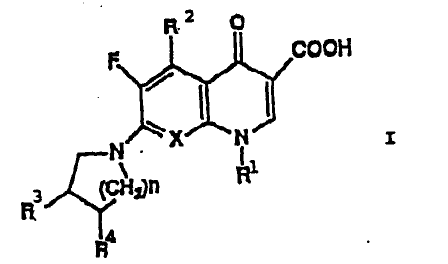 wherein R¹ is a C₃-C₆ cycloalkyl group or a phenyl group which may be substituted by methoxy or amino or by one or two halogen atoms; R² is hydrogen, a halogen atom, a C₁-C₄ alkyl group, a hydroxy group or an amino group; R³ is hydrogen, hydroxy or amino; R⁴ is a 1,2,3-triazol-1-yl group, a 1,2,4-triazol-1-yl group, a 1,2,3,4-tetrazol-1-yl group or a 1,2,3,4-tetrazol-2-yl group, each of which may have 1 to 2 substituents selected from the group consisting of C₁-C₄ alkyl, -COOH, -CH₂NH₂, amino and phenyl groups; and X is N, CH, C-F or C-OCH₃; n is 1; or 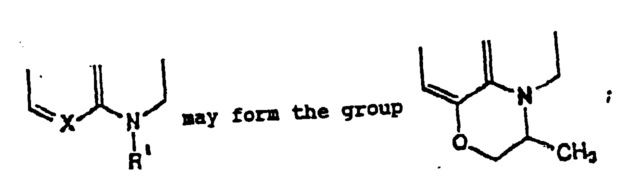 and pharmaceutically acceptable salts thereof.

Preferably the C₁-C₄ alkyl group is selected from methyl, ethyl, propyl, isopropyl, butyl, isobutyl or t-butyl; the C₃-C₆ cycloalkyl group is selected from cyclopropyl, cyclobutyl, cyclopentyl or cyclohexyl and the optionally substituted phenyl is selected from phenyl, 4-fluorophenyl, 2,4 -difluorophenyl, 2,4 -dimethoxyphenyl and 4-aminophenyl. The groups which may be substituted, as discussed above, may suitably be substituted with chlorine, bromine, fluorine, methoxy or amino.

The compounds of the invention include those wherein the pyrrolidine moiety at the 7-position has an asymmetric carbon atom or atoms and can be in optically active forms. Hence, this invention includes the R isomer, the S isomer and mixtures thereof. Some of the compounds of this invention have two asymmetric carbon atoms on the pyrrolidine moiety and therefore exist as stereoisomers having different configurations (i.e., cis-and transconfigurations). Such stereoisomers and their mixtures are also included within the compounds of this invention.

The compounds of this invention exhibit excellent antimicrobial activity against both sensitive and resistant Gram-positive bacteria. Compounds of formula I may be utilised as antibacterial active compounds in medicaments formulated with pharmaceutically acceptable carriers.

### Description of the Preferred Embodiments

In general, the 7-substituted-6-fluoro-1,4-dihydro-4-oxo-quinoline-3-carboxylic acid compounds of formula I are prepared as follows:

Compounds having the general formula II are reacted with compounds having the general formula III under the conditions described hereinafter. 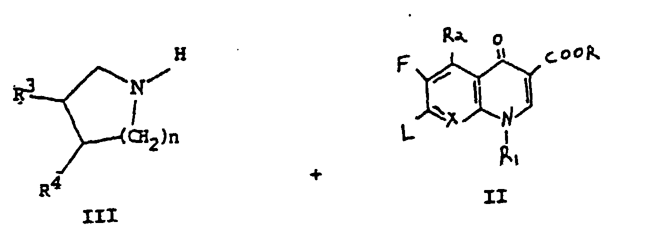 L is a suitable leaving group and may be chlorine, bromine, fluorine, SO₂R₇, wherein R₇ is a C₁-C₄ alkyl group or an unsubstituted or substituted phenyl group. The starting compounds having the formula II can be prepared from known starting materials using standard procedures or variations thereof within the skill of the art. Various starting compounds of formula II are known and are shown below in association with a reference citation: 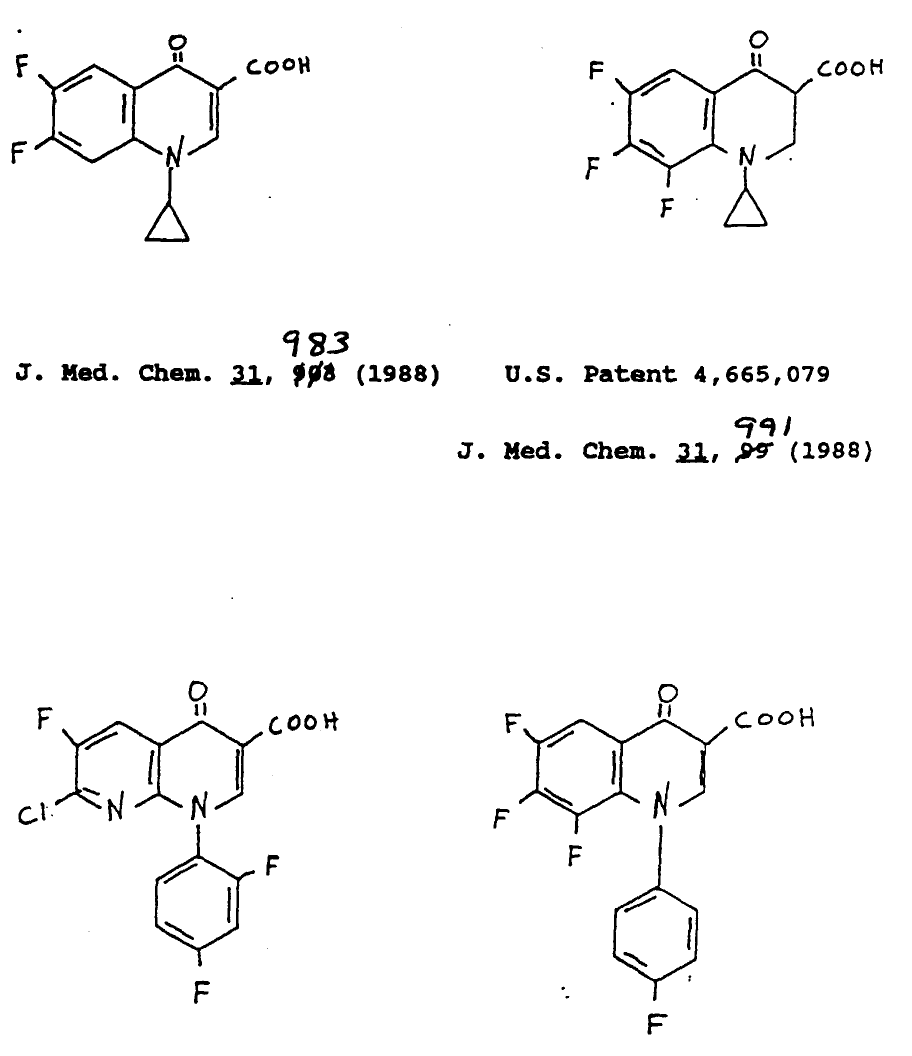 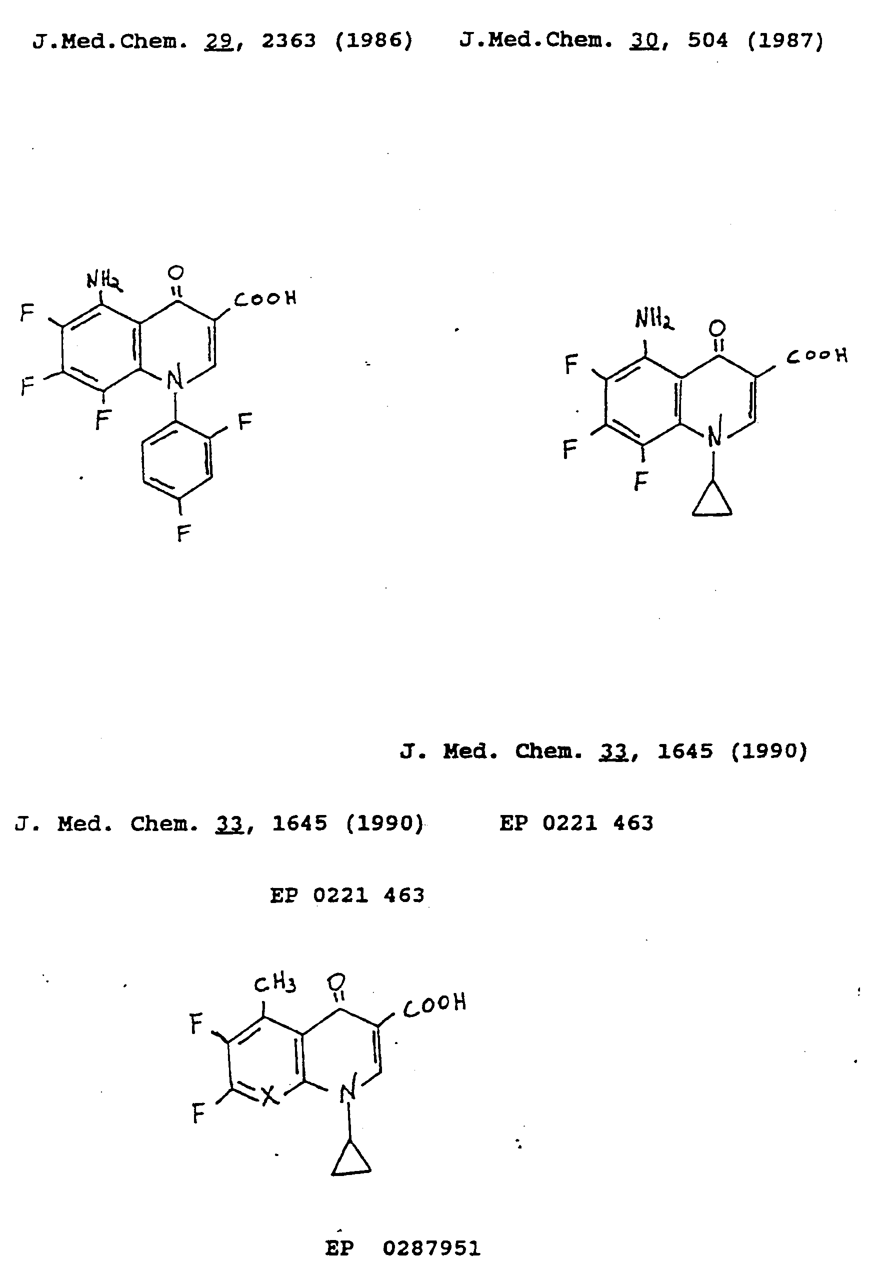 wherein X is nitrogen, or C-F or CH or C-CH₃ or C-CF₃ or C-OCH₃.

A schematic route for the preparation of compounds of formula III wherein the azole ring is a 1,2,3-triazole is given below: 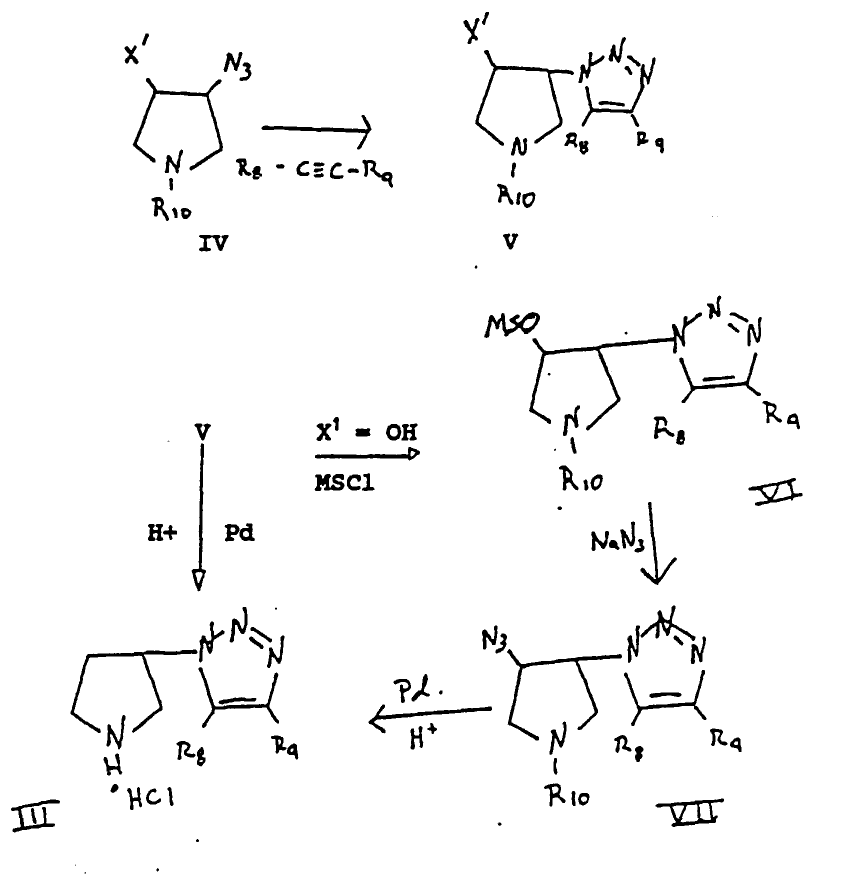 wherein X¹ is hydrogen or hydroxy,
R₃ is a hydrogen or hydroxy or amino group,
R₈ and R₉ are hydrogen C₁-C₄ alkyl, COOH, CH₂NH₂, amino or phenyl,
R₁₀ is a benzyl or t-BOC protective group.

The reaction of compound IV and a substituted acetylene as shown is carried out in a suitable solvent such as acetone, methanol, ethanol, benzene, toluene or xylene, either in a high pressure vessel or at normal pressure. The reaction is usually carried out at temperature from room temperature to 150°C, preferably from 60° C to 140° C, for 2 to 96 hours. Substituted acetylenes are usually used in an amount of at least 1 mole, preferably 1 to 15 moles, for every one mole of compound IV.

Deprotection of the N-protective group of compound V is carried out either by hydrogenation in presence of an acid such as hydrochloric acid, or acetic acid or by hydrolysis with mineral acid such as HC1, HNO₃, H₂SO₄, CF₃COOH or CH₃COOH in a solvent such as methanol, ethanol or propanol. The deprotection reaction is usually carried out at a temperature from 0° C to 100° C, usually at O° C to 40° C, for 10 minutes to 48 hours. The hydrogenation reaction is usually carried out in presence of metal catalyst such as Pd, Pt or Rh under normal pressure to high pressure.

The reaction of compound V and methane sulfonyl chloride (MSCl) is carried in a suitable solvent, for example, dichloromethane, chloroform, carbon tetrachloride, benzene, toluene, DMF or DMSO in the presence of a base such as triethylamine, NaHCO₃, K₂CO₃, CsCO₃, sodium alkoxide (NaOCH₃ or NaOC₂H₅), potassium tert-butoxide or pyridine. The reaction temperature is in the range from 0° C to 100° C, preferably 0° C to 35° C, and reaction times vary from 1 hour to 48 hours. The methanesulfonyl chloride is usually used in an amount of at least 1 mole preferably 1 to 5 moles per mole of compound V.

The reaction of compound VI and a metal azide such as NaN₃ in the presence of phase transfer catalyst such as NH₄C1, (NH₄)₂CO₃, (Bu)₄NBr is carried out in a polar solvent such as DMF or DMSO, or in a mixture of solvents such as DMF-H₂O, DMSO-H₂O in a ratio of 4:1. The reaction temperature ranges from room temperature to 180° C, preferably from 40° to 100°C. The reaction time ranges from 1 hour to 48 hours, and a molar ratio of from 1 to 5 moles of metal azide per mole of compound VI is preferred. The phase transfer catalysts are used in same ratio as the metal azide ratio.

The reaction condition for conversion of compound VII to compound III is the same as described for conversion of compound V to compound III.

Compounds of the formula III wherein the azole ring is a 1,2,4-triazole can be prepared by the reaction of potassium-1,2,4-triazolide with N-benzyl-3-mesyloxypyrrolidine. The N-blocking groups are then removed are in the usual manner by catalytic hydrogenation with palladium on charcoal, as illustrated in Examples R and S.

Compounds of the formula III wherein the azole ring is a 1,2,3,4-tetrazole moiety are prepared in an analogous manner as illustrated in Examples DD, EE and FF.

The starting compounds II and III are reacted together in the presence of solvents at elevated or reduced temperatures for a sufficient time to allow the reaction to proceed to completion. Reaction conditions will depend upon the nature of the leaving group L of the compounds of formula II and the degree of the reactivity of compound III.

The reaction is preferably carried out in the presence of a proton acceptor such as pyridine, diazabicycloundecane (DBU), N-methyl pyrrolidine-2-one or picoline.

The solvents of choice for this reaction are nonreactive solvents such as acetonitrile, tetrahydrofuran (THF), ethanol, methanol, chloroform, methylene chloride, pyridine, picoline, N-methylpyrrolidine-2-one, water, dimethyl sulfoxide and dimethylformamide. Mixtures of these solvents may also be utilised.

Reaction temperatures will generally range from between about 50°C to 150°C. The preferred molar ratio of compounds II and III are 1:2.5 to 5.0. The reaction times generally range from 4 to 50 hours depending on the reactants.

Another method for preparing compounds of formula I is by reacting a compound of the general formula II with a compound of formula VIII to form a compound of formula IX followed by substitution of leaving group L₁ in IX with the appropriately substituted azoles as shown below. 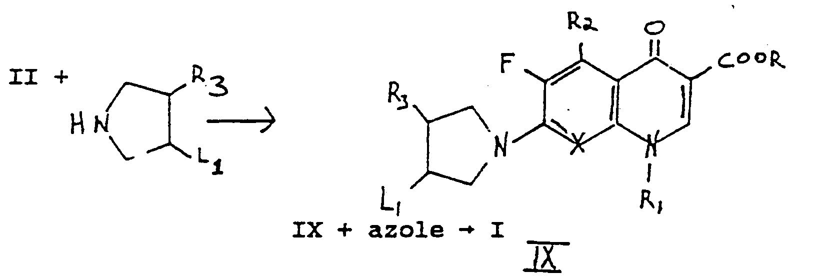 R₁, R₂, R₃, X and L₁ are the same as defined above. L₁ is the suitable leaving group selected from chlorine, bromine, fluorine or SO₂CH₃ or SO₂C₆H₄ CH₃(p).

An alternate process can also be used for preparing the compound of formula I by reacting the compound of formula II with compound of formula XI followed by reaction with substituted acetylenes as shown in the following scheme: 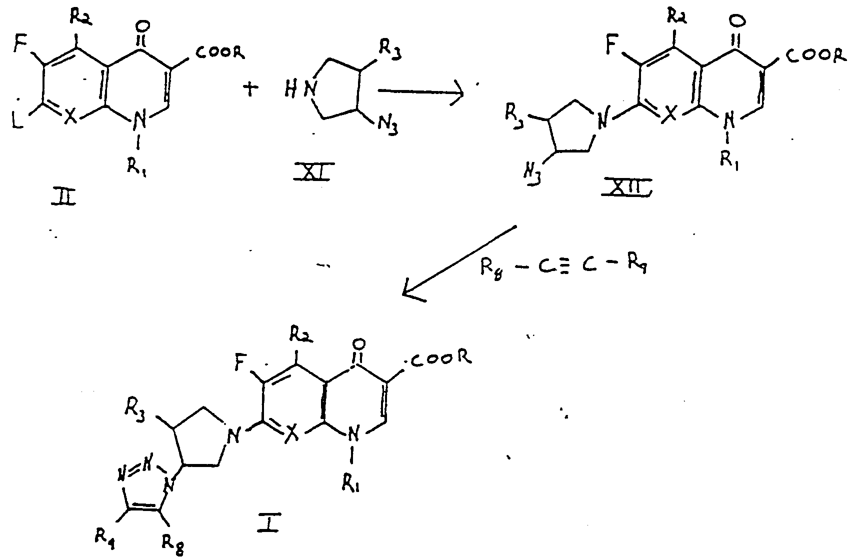 R₁, R₂, R₃, R₈, R_{q}, X and L are as defined above. The reaction time, temperature, molar ratio and solvent for the reaction of the compound of formula II with the compound of formula VIII or reaction of compound of formula II with compound of formula XI are the same as defined for the reaction of compound II with compound III, as described above.

The reaction temperature, time, molar ratio and solvent used for the reaction of compound IX with triazoles are the same as described above for the reaction of compound VI with metal azides NaN₃ or (C₄H₉)₄NN₃. The reaction temperature, times, molar ratio and solvent used for the reaction of compound XII with substituted acetylenes are the same as described for the conversion of compound IV to compound V.

The pyrido-benzoxazine compounds of the invention are prepared as illustrated in Examples 38, 39 and 40 using the procedure described by Mitscher et al, J. Med. Chem. 30, 2283-2286 (1987) to prepare the pyrido-benzoxazine intermediate compounds of formula II.

The compounds of the invention are capable of forming both pharmaceutically acceptable acid addition and/or base salts. Base salts are formed with alkali and alkaline earth metals such as sodium, potassium, magnesium and calcium and heavy metal salts such as silver, zinc, cobalt, and cerium, and with organic amines such as choline and lysine, either directly or in combination with a physiologically acceptable carrier.

Pharmaceutically acceptable acid addition salts are formed with organic and inorganic acids such as hydrochloric, sulphuric, phosphoric, acetic, citric, oxalic, malonic, salicylic, malic, gluconic, fumaric, succinic, ascorbic, maleic, methanesulfonic and p-toluenesulfonc acid. The salts are prepared by contacting the free base form with a sufficient amount of the desired acid or amine to produce, for example, the mono or di salt in a conventional manner.

The compounds of the invention can exist in unsolvated as well as solvated forms including hydrated forms. In general, the solvated forms, including hydrated forms, are equivalent to the unsolvated forms for purpose of the invention.

The compounds of formula I are useful as antibacterial agents. They were found to be very potent in vitro against various sensitive and quinoline resistant Gram positive microbes such as E. faecium. S. aureus Cog. -ve, S, epidermidis, S. saprophyticus, and S. pyogenes. Further studies on some of the compounds of this invention revealed that their in vitro MIC values against Gram-positive and Gram-negative organisms are negligibly affected by inoculum size, cations (Mg⁺⁺, Ca⁺⁺), and serum.

Human patients suffering from bacterial infections can be treated by administering to the patient a pharmaceutically effective amount of one or more of the present compounds optionally, but preferably, in the presence of a pharmaceutically acceptable carrier or diluent. There may also be included a pharmaceutically compatible binding agent, and/or ajuvant materials. The active materials can also be mixed with other active materials which do not impair the desired action and/or supplement the desired action. The above materials according to the present invention can be administered by any route, for example, orally, parenterally, intravenously, intradermally, subcutaneously or topically in solid or liquid form.

The solid form preparation includes powders, tablet, dispensable granules, capsules, cachets, suppositories and ointments. A solid carrier can be one or more substances which may also act as diluents, flavoring agents, solubilizers, lubricants, suspending agents, binders, or tablet disintegrating agents, including magnesium carbonate, magnesium stearate, talc, sugar, lactose, pectin, dextrin, starch, gelatin, gum tragacanth, methyl cellulose, sodium carboxymethyl cellulose, low melting wax and cocoa butter.

Liquid form preparations include solutions, suspensions and emulsions. The liquid preparation for parenteral injection may be water or water-propylene glycol solution or water-polyethylene glycol solution, the-like so long as it is acceptable to biological systems (isotonicity, pH etc.). Aqueous solutions suitable for oral use can be prepared by dissolving the active component in water and adding suitable colorant, flavors, stabilizing and thickening agents as desired. Aqueous suspensions suitable for oral use can be made by dispersing the finely divided active component in water with viscous material, i.e. natural or synthetic gum, resins, methyl cellulose or sodium carboxymethyl cellulose.

The quantities of active compound in a unit dose of a preparation may be varied depending on the particular application and the potency of the active ingredient. Determination of the proper dosage for a particular situation is within the skill of the art. The dosage of the pharmaceutical preparation is generally in the range of 0.2 to 100 mg of the compound of formula I and salts thereof per kilogram of body weight of the patient per day. Preferably this daily dose is administered 2 - 4 times per day in fractions of the daily dose.

Our invention is further illustrated by means of the following examples:

### Example 1

### Ethyl 6,8-difluoro-1-(4-fluorophenyl)-7-[3-(1,2,3-triazol-1-yl)pyrrolidin-1-yl]-1,4-dihydro-4-oxo-quinoline-3-carboxylate

A solution of ethyl 1-(4-fluorophenyl)-6,7,8-trifluoro-1,4-dihydro-4-oxo-quinoline-3-carboxylate (180 mg, 0.5 mmol), 3-(1,2,3-triazol-1-yl)-pyrrolidine hydrochloride (259 mg, 1.4 mmol) and DBU (384 mg, 2.5 mmol) in acetonitrile (20 ml) was heated at 75°C for 3 h. Cooled to r.t. and stirred for another 15 h. The separated solid was filtered, successively washed with acetonitrile and ether. The white crystalline solid thus obtained was dried in vac-oven at 40°C. Yield: 135 mg. 56.7%. ¹H NMR (CDCl₃) δ: 1.35 (t, 3H), 2.50 (m, 2H), 3.70 (m, 1H), 3.85 (m, 2H), 4.10 (m, 1H), 4.35 (q, 2H), 5.25 (m, 1H), 7.20 (m, 2H), 7.40 (m, 2H), 7.63 (d, 1H), 7.72 (d, 1H), 7.90 (dd, 1H), 8.25 (S, 1H).

### Example 2

### 6,8-Difluoro-1-(4-fluorophenyl)-7-[3-(1,2,3-triazol-1-yl)-pyrrolidin-1-yl]-1,4-dihydro-4-oxo-quinoline-3-carboxylic acid

A suspension of ethyl 6,8-difluoro-1-(4-fluorophenyl-7-[3-(1,2,3-triazol-1-yl)-pyrrolidin-1-yl]-1,4-dihydro-4-oxo-quinoline-3-carboxylate (180 mg, 0.37 mmol) in 20 ml NaOH solution (containing 15 mg NaOH) and THF (20 ml) was heated at 90° C for 3.5 h. The THF was evaporated and the separated solid redissolved in water layer by heating and acidified to pH 6.0. The precipitate was filtered, washed with water and dried in vac-oven at 40° C to obtain the title compound as a light yellow solid. Yield: 89 mg, 52.66%; m.p. 303° C. ¹H NMR (TFA) δ: 2.82 (m, 2H), 4.14-4.64 (m, 4H), 5.74 (m, 1H), 7.37 (m, 2H), 7.60 (m, 2H), 8.20 (dd, 1H), 8.54 (d, 1H), 8.65 (d, 1H), 9.03 (S, 1H).

### Example 3

### Ethyl 1-(2,4-difluorophenyl)-6-fluoro-7-[3-(1,2,3-triazol-1-yl)-pyrrolidin-1-yl]-1,4-dihydro-4-oxo-1,8-naphthyridine-3-carboxylate

A mixture of ethyl 1-(2,4-difluorophenyl)-7-chloro-6-fluoro-1,4-dihydro-4-oxo-1,8-naphthyridine-3-carboxylate (91 mg, 0.5 mmol), 3-(1,2,3-triazol-1-yl)-pyrrolidine hydrochloride (259 mg, 1.5 mmol), and DBU (380 mg, 2.5 mmol) in CH₃CN (20 ml) was heated under reflux for 2 h, cooled to r.t. and stirred further for 18 h, diluted with water. Unreacted starting materials were removed by extraction with chloroform. The water layer was concentrated to give a yellow oil. Yield: 150 mg, (62%). ¹H NMR (CDCL₃) δ: 1.3 (t, 3H), 2.5 (m, 2H), 3.9 (m, 4H), 4.4 (q, 2H), 5.3 (m, 1H), 7.3 (m, 4H), 7.8 (d, 1H), 8.0 (d, 1H), 8.4 (s, 1H).

### Example 4

### Ethyl 1 -cyclopropyl-6-fluoro-7-[3(S)-(1,2,3-triazol-1-yl)pyrrolidin-1-yl]-1,4-dihydro-4-oxo-1,8-naphthyridine-3-carboxylate

Ethyl 7-chloro-1-cyclopropyl-6-fluoro-1,4-dihydro-4-oxo-1,8-naphthyridine-3-carboxylate (250 mg, 0.8 mmole) was reacted with 350 mg (2 mmole) of 3(S)-(1,2,3-triazol-1-yl)pyrrolidine hydrochloride in 8 ml of pyridine in the presence of 305 mg (2 mmol) of DBU at 80-90°C for 6 hrs. The reaction was then stirred at room temperature for 4 days. The solvent was then evaporated under reduced pressure and to the residue, water was added and extracted with chloroform. The organic layer was dried and evaporated to dryness. The residue was then chromatographed over alumina (neutral, activity III) using chloroform as solvent to yield 80 mg (24%) of the desired product. ¹H NMR (CDCL₃) δ: 8.48 (s, 1H), 8.05 (d, 1H), 7.76 (d, 1H), 7.71 (d, 1H), 5.46-5.32 (m, 1H), 4.46-4.26 (m, 4H), 4.15-4.0 (m, 2H), 3.56-3.42 (m, 1H), 2.71-2.57 (m, 2H), 1.4 (t, 3H), 1.26-0.95 (m, 4H).

### Example 5

### 1-Cyclopropyl-6-fluoro-7-[3(S)-(1,2,3-triazol-1-yl)pyrrolidin-1-yl[-1,4-dihydro-4-oxo-1,8-naphthyridine-3-carboxylic acid

Ethyl 1-cyclopropyl-6-fluoro-7-[3(S)-(1,2,3-triazol-1-yl)pyrrolidin-1-yl]-1,4-dihydro-4-oxo-1,8-naphthyridine-3-carboxylate (80 mg, 0.19 mmol) was heated in 16 ml of 6N HCl at 100-110°C for 18 hrs. This was then concentrated to dryness and methanol-ether was added and the formed solid was collected to yield 55 mg (73%) of the desired product after drying, m.p. 268-270° C. ¹H NMR (TFA) δ : 9.21 (s, 1H), 8.74 (s, 1H), 8.58 (s, 1H), 8.20 (d, 11.4 Hz, 1H), 6.14-5.84 (m, 1H), 4.94-4.3 (m, 4H), 4.18-3.95 (m, 1H), 3.18-2.8 (m, 2H), 1.68-1.18 (m, 4H).
Anal. calcd. for C₁₈H₁₇FN₆O₃·1/2H₂O; C, 54.96; H, 4.61; N, 21.35. Found; C, 54.66; H, 4.51; N, 20.85.

### Example 6

### Ethyl 7-[cis-3-amino-4-(1,2,3-triazol-1-yl)pyrrolidin-1-yl] -1-cyclopropyl-6-fluoro-1,4-dihydro-4-oxo-1,8-naphthyridine-3-carboxylate

Ethyl 7-chloro-1-cyclopropyl-6-fluoro-1,4-dihydro-4-oxo-1,8-naphthyridine-3-carboxylate (243 mg, 0.78 mmole) and cis-3-amino-4-(1,2,3-triazol-1-yl) pyrrolidine (300 mg, 1.96 mmole) were reacted together in 8 ml of pyrridine at room temperature for 5 days. The solvent was then evaporated, water was added to the residue and was extracted with chloroform. The organic layer was then dried (Na₂SO₄) and evaporated to yield 0.23 g of the crude product which upon purification over neutral alumina (activity III) using 4% methanol/chloroform as solvent yielded 200 mg (60%) of the desired product. ¹H NMR (CDCl₃) δ: 8.5 (s, 1H), 8.12 (d, 1H), 7.82 (s, 1H), 7.76 (s, 1H), 5.3-5.15 (s, 1H), 4.55-4.00 (m, 6H), 3.85-3.62 (m, 1H), 3.58-3.4 (m, 1H), 1.43 (t, 3H), 1.32-0.94 (m, 4H).

### Example 7

### 7-[cis-3-Amino-4-(1,2,3-triazol-1-yl)pyrrolidin-1-yl]-1-cyclopropyl-6-fluoro-1,4-dihdyro-4-oxo-1,8-naphthyridine-3-carboxylic acid hydrochloride

Ethyl 7-[cis-3-amino-4-(1,2,3-triazol-1-yl)pyrrolidin-1-yl] -1-cyclopropyl-6-fluoro-1,4-dihydro-4-oxo-1,8-naphthyridine-3-carboxylate (200 mg, 0.47 mmole) was heated with 8 ml of 6N hydrochloric acid at 110°C for 18 hrs. The solution was then evaporated to dryness and the residue was crystallized from methanol-ether to yield 160 mg (85%) of the desired hydrochloride, m.p. 240° C (dec). ¹H NMR (TFA) δ : 9.27 (s, 1H), 9.01 (s, 1H), 8.66 (s, 1H), 8.3 (d, 11.6 Hz, 1H), 6.64-6.45 (m, 1H), 5.35-4.66 (m, 5H), 4.20-4.02 (m, 1H), 1.7-1.2 (m, 4H).Anal. calcd. for C₁₈H₁₉ClFN₇O₃.1½H₂O C, 46.76; H, 4.80; N, 21.2; Cl, 7.67. Found; C, 46.85; H, 4.31; N, 20.62; Cl, 8.36.

### Example 8

### 1-(2,4-Difluorophenyl)-6-fluoro-7-[3-(1,2,3-triazol-1-yl)pyrrolidin-1-yl]-1,4-dihydro-4-oxo-1,8-naphthyridine-3-carboxylic acid

A mixture of crude ethyl 1-(2,4-difluorophenyl)-6-fluoro-7-[3-(1,2,3-triazoi-1-yl)-pyrrolidin-1-yl]-1,4-dihydro-4-oxo-1,8-naphthyridine-3-carboxylate (150mg, 0.31 mmol), THF (15 ml), NaOH (20 mg, 1.5 mmol) and water 15 ml was heated under reflux for 3 h, cooled and the THF evaporated. The aqueous solution was acidified and the precipitated yellow solid washed with water, dried in vac-oven at 50° C to obtain 80 mg (solid). This solid was washed with ether to get the pure of compound. Yield: 50 mg (35.7%) m.p.: 253° C (dec.). ¹H NMR (TFA) δ: 2.85 (m, 2H), 3.70-4.50 (m, 4H), 5.80 (m, 1H), 7.20 (m, 2H), 7.60 (m, 1H), 8.25 (s, 1H), 8.57 (S, 1H), 8.62 (S, 1H), 9.20 (S, 1H).

### Example 9

### 1-Cyclopropyl-6-fluoro-7-(3-(1,2,3-triazol-1-yl)pyrrolidin-1-yl]-1,4-dihydro-4-oxo-quinoline-3-carboxylic acid

A mixture of 1-cyclopropyl-6,7-difluoro-1,4-dihydro-4-oxo-quinoline-3-carboxylic acid (120 mg, 0.486 mmol), DBU (190 mg, 1.25 mmol),3-(1,2,3-triazol-1-yl)pyrrolidine hydrochloride (216 mg, 1.25 mmol) in pyridine (8 ml) was heated at 80° C for 18 h. The reaction mixture was concentrated and treated with water. The separated solid was collected by filtration, washed with water and acetonitrile to give light brown solid. Yield: 71 mg (38%), m.p. 284° C (dec). ¹H NMR (TFA) δ: 1.5 (m, 4H), 3.0 (m, 2H), 4.0 (m, 4H), 4.65 (bs, 1H), 5.9 (bs, 1H), 7.5 (d, 1H), 8.20 (d, 1H), 8.6 (s, 1H), 8.7 (s, 1H), 9.2 (s, 1H).

### Example 10

### 1-Cyclopropyl-6,8-difluoro-7-[3-(1,2,3-triazol-1-yl)pyrrolidin-1-yl]-1,4-dihydro-4-oxo-quinoline-3-carboxylic acid

A mixture of 1-cyclopropyl-6,7,8-trifluoro-1,4-dihydro-4-oxo-quinoline-3-carboxylic acid (138.5 mg, 0.486 mmol), DBU (190 mg, 1.25 mmol), and 3-(1,2,3-triazol-1-yl)-pyrrolidine hydrochloride (216 mg, 1.25 mmol) in pyridine (8 ml) was heated for 20 h at 75-80° C and then pyridine removed under vacuum. The residue was diluted with acetonitrile. The precipitated solid was washed with acetonitrile, ether and dried at 40° C. Yield: 65 mg (33%) m.p. 244-246° C (dec.). ¹H NMR 25 (TFA) δ:1.5 (m, 4H), 2.8 (m, 2H), 4.5 (m, 4H), 4.75 (bs, 1H), 5.8 (bs, 1H), 8.15 (d, 1H), 8.6 (s, 1H), 8.7 (s, 1H), 9.25 (s, 1H).

### Example 11

### 5-Amino-1-cyclopropyl-6,8-difluoro-7-[3-(1,2,3-triazol-1-yl)-pyrrolidin-1-yl]-1,4-dihydro-4-oxo-quinoline-3-carboxylic acid

A mixture of 5-amino-1-cyclopropyl-6,7,8-trifluoro-1,4-dihydro-4-oxo-quinoline-3-carboxylic acid (190 mg, 0.64 mmol),3-(1,2,3-triazol-1-yl)-pyrrolidine hydrochloride (290 mg, 1.67 mmol), and DBU (255 mg, 1.70 mmol) in pyridine (5 ml) was heated at 110° C for 20 h. The reaction mixture was then cooled and diluted with methanol, the separated solid was collected and washed successively with methanol, water and acetonitrile. The title compound was obtained upon drying in oven at 40° C. Yield: 168 mg (63%) m.p.: 273.5-275° C. ¹H NMR (TFA) δ: 1.40 (m, 4H), 2.90 (m, 1H), 4.40 (m, 4H), 4.75 (m, 1H), 5.70 (m, 1H), 8.55 (d, 1H), 8.68 (d, 1H), 9.15 (S, 1H).

### Example 12

### 5-Amino-1-cyclopropyl-6,8-difluoro-7-[3S-(1,2,3-triazol-1-yl)-pyrrolidin-1-yl]-1,4-dihydro-4-oxo-quinoline-3-carboxylic acid

A mixture of 5-amino-1-cyclopropyl-6,7,8-trifluoro-1,4-dihydro-4-oxo-quinoline-3-carboxylic acid (200 mg, 0.67 mmol, 3S-(1,2,3-triazol-1-yl)pyrrolidine hydrochloride (293 mg,1.68 mmole) and diazabicycloundecane (256 mg, 1.60 mmol) in pyridine (20 ml) was heated at 110° C for 20 hrs. The reaction mixture was cooled, diluted with methanol, the separated solid was filtered and washed with H₂0 and CH₃CN, dried to give title product. Yield: 120 mg (43%), m.p. 277-78° C. ¹H NMR (TFA) δ: 1.31-1.58 (m, 4H), 2.90 (m, 2H), 4.15-4.54 (m, 4H), 4.75 (m, 1H), 5.86 (m, 1H), 8.55 (d, 1H), 8.69 (d, 1H), 9.15 (S, 1H).

### Example 13

### 5-Amino-1-cyclopropyl-6,8-difluoro-7-[3R-(1,2,3-triazol-1-yl)-pyrrolidin-1-yl)-1,4-dihydro-4-oxo-quinoline-3-carboxylic acid

Prepared by the same procedure as described in Example 12 by using 5-amino-1-cyclopropyl-6,7,8-trifluoro-1,4-dihydro-4-oxo-quinoline-3-carboxylic acid and 3R-(1,2,3-triazol-1-yl) pyrrolidine hydrochloride. Yield 41%, m.p. 279-280° C. ¹H NMR (TFA) δ: 1.31-1.49 (m, 4H), 2.92 (m, 2H), 4.2-5.1(m, 4H), 4.73 (m, 1H), 5.82 (m, 1H), 8.56 (d, 1H), 8.68 (d, 1H), 9.10 (s, 1H).

### Example 14

### 5-Amino-1-(2,4-difluorophenyl)-6,8-difluoro-7-[3-(1,2,3-triazol-1-yl)pyrrolidin-1-yl]-1,4-dihydro-4-oxo-quinoline-3 carboxylic acid

A mixture of 5-amino-1-(2,4-difluorophenyl)-6,7,8-trifluoro-1,4-dihydro-4-oxo-quinoline-3-carboxylic acid (200 mg, 0.54 mmol), 3-(1,2,3-triazol-1-yl)pyrrolidine hydrochloride (231 mg, 1.25 mmol) and diazabicycloundecane (200 mg, 1.31 mmol) in acetonitrile (25 ml) was refluxed for 45 hrs. The separated solid was filtered, washed with acetonitrile and ether to give 150 mg (57%) of desired product, m.p. 307.5-309° C. ¹H NMR (TFA) δ: 2.81 (m, 2H), 4.06-4.53 (m, 4H), 5.73 (m, 1H), 7.15-7.23 (m, 2H), 7.59-7.66 (m, 1H), 8.52 (d, 1H), 8.62 (d, 1H), 8.82 (d, 1H).

### Example 15

### 5-Amino-1-(2,4-difluorophenyl)-6,8-difluoro-7-[3S-(1,2,3-triazol-1-yl)-pyrrolidin-1-yl-1,4-dihydro-4-oxo- quinoline-3-carboxylic acid

A mixture of 5-amino-1-(2,4-difluorophenyl)-6,7,8-trifluoro-1,4-dihydro-4-oxo-quinoline-3-carboxylic acid (100 mg, 0.27 mmol), 3S-(1,2,3-triazol-1-yl)pyrrolidine hydrochloride (117 mg, 0.67 mmol) and diazabicycloundecane (102 mg, 0.67 mmol) in pyridine (10 ml) was heated at 110° C for 40 hrs. The reaction mixture was concentrated and the residue was triturated with water. The separated solid was filtered, washed with water, acetonitrile and dried under vacuum at 40° C. Yield: 88 mg (67%); m.p. 302-304° C. ¹H NMR (TFA) δ: 2.82 (m, 2H), 4.0-4.53 (m, 4H), 5.73 (m, 1H), 7.10-7.23 (m, 2H), 7.55-7.70 (m, 1H)8.52 (d, 1H), 8.62 (d, 1H), 8.82 (d, 1H).

### Example 16

### 5-Amino-1-(2,4-difluorophenyl)-6,8-difluoro-7-[3R-(1,2,3-triazol-1-yl)-pyrrolidin-1-yl]-1,4-dihydro-4-oxo-quinoline-3-carboxylic acid

Prepared by following the procedure as described in Example 15 by using 5-amino-1-(2,4-difluorophenyl)-6,7,8-trifluoro-1,4-dihydro-4-oxo-quinoline-3-carboxylic acid and 3R-(1, 2, 3-triazol-1-yl) pyrrolidine hydrochloride. Yield: 57%, m.p. 302.5-304°C. ¹H NMR (TFA) δ: 2.80-2.90 (m, 2H), 4.05-4.35 (m, 3H), 4.55 (m, 1H), 5.73 (m, 1H), 7.09-7.23 (m, 2H), 7.55-7.70 (m, 1H), 8.53 (d, 1H), 8.62 (d, 1H), 8.82 (d, 1H).

### Example 17

### 7-[cis-3-Amino-4-(1,2,3-triazol-1-yl)-pyrrolidin-1-yl]-1-cyclopropyl-6,8-difluoro-1,4-dihydro-4-oxo-quinoline-3-carboxylic acid

A mixture of 1-cyclopropyl-6,7,8-trifluoro-1,4-dihydro-4-oxo-quinoline-3-carboxylic acid (44 mg, 0.156 mmol) and cis-3-amino-4-(1,2,3-triazol-1-yl)pyrrolidine (60 mg, 0.39 mmol) in pyridine (5 ml) was heated at 110° C for 2-3 hrs under N₂ and then stirred at room temperature for 20 hrs. The reaction mixture was concentrated and the residue was washed with water and acetonitrile. The solid was dried under vacuum at 40° C. Yield: 43 mg (66%), m.p. 245-47° C. ¹H NMR (TFA) δ: 1.3-1.8 (m, 4H), 4.4-5.25 (m, 6H), 6.40 (m, 1H), 8.15 (d, 1H), 8.62 (S, 1H), 8.90 (S, 1H), 9.40 (S, 1H).

### Example 18

### 5-Amino-7-[cis-3-amino-4-(1,2,3-triazol-1-yl)-pyrrolidin-1-yl]-1-cyclopropyl-6,8-difluoro-1,4-dihydro-4-oxo-quinoline-3-carboxylic acid

Prepared by following the same procedure as described for Example 17 by using 5-amino-1-cyclopropyl-6,7,8-trifluoro-1,4-dihydro-4-oxo-quinoline-3-carboxylic acid and cis 3-amino-4-(1,2,3-triazol-1-yl) pyrrolidine. Yield: 65%, m.p. 275-277° C. ¹H NMR (TFA) δ: 1.33-1.50 (m, 4H), 4.32-5.12 (m, 6H), 6.34 (m, 1H), 8.64 (S, 1H), 8.89 (S, 1H), 9.16 (S, 1H).

### Example 19

### 5-Amino-7-[cis-3-amino-4-(1,2,3-triazol-1-yl)-pyrrolidin-1-yl]-1-(2,4-difluorophenyl)-6,8-difluoro-1,4-dihydro-4-oxo-quinoline-3-carboxylic acid

Prepared by following the same procedure as described for example 13 by using 5-amino-1-(2,4-difluorophenyl)-6,7,8-trifluoro-1,4-dihydro-4-oxo-quinoline-3-carboxylic acid and cis 3-amino-4-(1,2,3-triazol-1-yl)-pyrrolidine. Yield: 66%, m.p. 279-281° C. ¹H NMR (TFA) δ: 4.48-4.82 (m, 4H), 5.02 (m, 1H), 6.26 (m, 1H), 7.17 (m, 2H), 7.62 (m, 1H), 8.6 (S, 1H), 8.82 (2d, 2H).

### Example 20

### 5-Amino-7-[trans-3-hydroxy-4-(1,2,3-triazol-1-yl)-pyrrolidin-1-yl]-1-cyclopropyl-6,8-difluoro-1,4-dihydro-4-oxo-quinoline-3-carboxylic acid

A mixture of 5-amino-1-cyclopropyl-6,7,8-trifluoro-1,4-dihydro-4-oxo-quinoline-3-carboxylic acid (63 mg, 0.21 mmol) trans 3-hydroxy-4-(1,2,3-triazol-1-yl)-pyrrolidine hydrochloride (100 mg, 0.52 mmol) and diazabicycloundecane (79 mg, 0.52 mmol) in pyridine was heated at 110°C for 20 hrs. The reaction mixture was concentrated and the residue was triturated with water. The separated solid was filtered, washed with water and acetonitrile and dried under vacuum at 40°C. Yield: 35 mg (40%); m.p. 261-165.5°C. ¹H NMR (TFA) δ : 1.33-1.51 (m, 4H) , 4.24 (m, 2H) , 4.60 (m, 2H), 4.92 (m, 1H) , 5.20 (m, 1H), 5.79 (m, 1H), 8.59 (d, 1H), 8.78 (s, 1H), 9.15 (s, 1H).

### Example 21

### 1-Cyclopropyl-6,8-difluoro-5-methyl-7-[3-(4-methyl-1,2,3-triazol-1-yl)pyrrolidin-1-yl]-1,4-dihydro-4-oxo-quinoline-3-carboxylic acid

To the suspension of 50 mg (0.168 mmole) of 1-cyclopropyl-5-methyl-6,7,8-trifluoro-1,4-dihydro-4-oxo-quinoline-3-carboxylic acid and 3-(4-methyl-1,2,3-triazol-1-yl)pyrrolidine hydrochloride (79 mg, 0.421 mmole) in dry acetonitrile (5 ml) was added 64 mg (0.421 mmole) of DBU (diazabicycloundecane) and the solution was refluxed under nitrogen for 46 hrs. The yellow solution was concentrated to dryness and the residue was then suspended in acetonitrile and filtered. The supernatant was evaporated to dryness and to the residue water was added and solid collected (30 mg, 43%), m.p. 232-234°C. ¹H NMR (TFA): 9.26 (s, 1H), 8.37 (s, 1H), 5.78-5.58 (m, 1H), 4.85-4.1 (m, 5H), 3.0-2.7 (m, 5H), 2.68 (s, 3H), 1.65-1.15 (m, 4H). Anal. calcd. for C₂₁H₂₁F₂N₅O₃: C, 58.74; H, 4.93; N, 16.31. Found: C, 58.14; H, 5.06; N, 16.08.

### EXAMPLE 22

### 1-Cyclopropyl-6,8-difluoro-7-[3-(5-methyl-1,2,3-triazol-1-yl)pyrrolidin-1-yl]-1,4-dihydro-4-oxo-quinoline-3-carboxylic acid

To 100 mg (0.36 mmole) of 1-cyclopropyl-6,7,8-trifluoro-1,4-dihydro-4-oxo-quinoline-3-carboxylic acid and 166 mg (0.9 mmole) of 3-(5-methyl-1,2,3-triazol-1-yl) pyrrolidine hydrochloride in 10 ml of pyridine was added 134 mg (0.9 mmole) of DBU and the resulting solution was heated under nitrogen at 120°C for 3 days. The very fine suspension was then filtered and evaporation of the supernatant afforded a residue which was crystallized upon addition of water. The solid was collected (117 mg), m.p. 199-200°C. ¹H NMR (CDCl₃) : 8.73 (s, 1H), 7.83 (d, 13.7 Hz, 1H), 7.5 (s, 1H), 5.1-4.9 (m, 1H), 4.48-3.88 (m, 5H), 2.77-2.45 (m, 2H), 2.4 (s, 3H), 1.4-1.1 (m, 4H). Anal. calcd. for C₂₀H₁₉F₂N₅O₃H₂₀; C, 55.42; H, 4.88; N, 16.16; Found; C, 55.64; H, 4.49; N, 15.73.

### Example 23

### 1-Cyclopropyl-6,8-difluoro-5-methyl-7-[3-(1,2,4-triazol-1-yl)pyrrolidin-1-yl]-1,4-dihydro-4-oxo-quinoline-3-carboxylic acid

1-Cyclopropyl-5-methyl-6,7,8-trifluoro-1,4-dihydro-4-oxo-quinoline-3-carboxylic acid (30 mg, 0.1 mmole) was reacted with 40 mg (0.2 mmole) of 3-(1,2,4-triazol-1-yl)pyrrolidine hydrochloride in 2 ml of pyridine in the presence of 70 mg (0.45 mmole) of DBU at 95°C overnight. The orange solution was evaporated to dryness and to the residue water was added. The orange solid was collected and washed with methanol to yield 19 mg of the crude product, which upon further purification from methanol yielded 12 mg of the desired product, m.p. 207-210°C. ¹H NMR (TFA) : 9.81 (s, 1H), 9.27 (s, 1H), 8.78 (s, 1H), 5.75-5.55 (m, 1H), 4.83-4.12 (m, 5H), 3-2.7 (m, 5H), 1.7-1.2 (m, 4H). Anal. calcd. for C₂₀H₁₉F₂N₅O₃·H₂O; C, 55.43; H, 4.88; N, 16.16; Found; C, 55.72; H, 4.73; N, 15.65.

### Example 24

### 1-Cyclopropyl-6,8-difluoro-5-methyl-7-[3-(1,2,3-triazol-1-yl)pyrrolidin-1-yl]-1,4-dihydro-4-oxo-quinoline-3-carboxylic acid

1-Cyclopropyl-5-methyl-6,7,8-trifluoro-1,4-dihydro-4-oxo-quinoline-3-carboxylic acid (30 mg, 0.1 mmole) was reacted with 35 mg (0.2 mmole) of 3-(1,2,3-triazol-1-yl)pyrrolidine hydrochloride in 2 ml of dry pyridine under nitrogen in the presence of 30.4 mg (0.2 mmole) of DBU under reflux conditions. The solvent was removed under reduced pressure and to the orange viscous oil water was added and the solid was collected which was further purified from methanol to yield 20 mg of the desired product, m.p. 228-230°C. ¹H NMR (TFA) : 9.28 (s, 1H), 8.68 (s, 1H), 8.57 (d, 1.4 Hz, 1H), 5.9-5.75 (m, 1H), 4.88-4.14 (m, 5H), 3.1-2.72 (m, 5H), 1.7-1.2 (m, 4H).

### Example 25

### 5-Amino-1-cyclopropyl-6,8-difluoro-7-[3-(4-methyl-1,2,3-triazol-1-yl)pyrrolidin-1-yl]-1,4-dihydro-4-oxo-quinoline-3-carboxylic acid

5-Amino-1-cyclopropyl-6,7,8-trifluoro-1,4-dihydro-4-oxo-quinoline-3-carboxylic acid (298 mg, 1 mmole) was reacted with 470 mg (2.5 mmole) of 3-(4-methyl-1,2,3-triazol-1-yl)pyrrolidine hydrochloride in the presence of 380 mg (2.5 mmole) of DBU in 20 ml of dry acetonitrile for two days under reflux conditions under the atmosphere of nitrogen. The yellow suspension was then evaporated to dryness and to the residue water was added. The yellow solid was filtered and crystallized repeatedly (3x) with acetonitrile to free the product from the unreacted starting material. After drying, 200mg of the desired product was obtained, m.p. 271-272.5°C. ¹H NMR (TFA) : 9.15 (s, 1H), 8.4 (s, 1H), 5.8-5.6 (m, 1H), 4.8-4.05 (m, 5H), 3.0-2.7 (m, 2H), 2.67 (s, 3H), 1.65-1.2 (m, 4H). Anal. calcd. for C₂₀H₂₀F₂N₆O₃·1/2 H₂O; C, 54.67; H, 4.82; N, 19.12; Found; C, 54.91; H, 4.69; N, 18.76.

### Example 26

### 1-Cyclopropyl-6,8-difluoro-7[3-(4,5-dimethyl-1,2,3-triazol-1-yl)pyrrolidin-1-yl]-1,4-dihydro-4-oxo-quinoline-3-carboxylic acid

1-Cyclopropyl-6,7,8-trifluoro-1,4-dihydro-4-oxo-quinoline-3-carboxylic acid (100 mg, 0.36 mmole) was reacted with 3-(4,5-dimethyl-1,2,3-triazol-1-yl)pyrrolidine hydrochloride (182 mg, 0.9 mole) in 8 ml of dry pyridine in the presence of 137 mg (0.9 mmole) of DBU at 115°C for five days. The small amounts of the solid was filtered off and the supernatant was evaporated to dryness. To the residue water and few drops of acetonitrile was added. The solid was collected to afford after drying 125 mg (81%) of the desired product as a gray solid, m.p. 230-232°C (dec). ¹H NMR (CDCl₃) : 8.74 (s, 1H), 7.84 (d, J=13.2 Hz, 1H), 5.07-4.8 (m, 1H), 4.4-3.85 (m, 5H), 2.8-2.14 (m, 8H, singlet at 2.29, 6H), 1.4-1.0 (m, 4H).
Anal Calcd. for C₂₁H₂₁F2N₅O₃·1/2 H₂O; C, 57.52; H, 4.83; N, 15.97; Found; C, 57.68; H, 4.81; 15.68.

### Example 27

### 1-Cyclopropyl-6,8-difluoro-7-[3-(4-methyl-1,2,3-triazol-1-yl)pyrrolidin-1-yl]-1,4-dihydro-4-oxo-quinoline-3-carboxylic acid

1-Cyclopropyl-6,7,8-trifluoro-1,4-dihydro-4-oxo-quinoline-3-carboxylic acid (150 mg, 0.53 mole) was reacted with 249 mg (1.33 mmole) of 3-(4-methyl-1,2,3-triazol-1-yl)pyrrolidine hydrochloride in the presence 201 mg (1.33 mmole) of DBU in 15 ml of dry pyridine under nitrogen at 120°C for 3 days. Small amounts of the solid present were removed by filtration, and the supernatant was concentrated under reduced pressure. Water and small amounts of acetonitrile were added to the residue and the grey solid was collected and dried to afford 180 mg (82%) of the desired product, m.p. 219-220°C. ¹H NMR (CDCl₃) : 8.73 (s, 1H), 7.85 (dd, J=13.7, 1.9 Hz, 1H), 7.42 (s, 1H), 5.4-5.3 (m, 1H), 4.4-3.7 (m, 5H), 2.7-2.5 (m, 2H), 2.37 (s, 3H), 1.4-1.05 (m, 4H). Anal. calcd. for C₂₀H₁₉F₂N₅O₃; C, 57.83; H, 4.57; N, 16.86; Found; C, 57.81; H, 4.60; N, 16.31.

### Example 28

### 5-Amino-1-cyclopropyl-6,8-difluoro-7-[3-(1,2,4-triazol-1-yl)pyrrolidin-1-yl]-1,4-dihydro-4-oxo-quinoline-3-carboxylic acid

To a suspension of 60 mg (0.2 mmole) of 5-amino-1-cyclopropyl-6,7,8-trifluoro-1,4-dihydro-4-oxo-quinoiline-3-carboxylic acid and 80 mg of 3-(1,2,4-triazol-1-yl)pyrrolidine hydrochloride in 3 ml of dry pyridine under N₂ was added 140 mg (0.9 mmole) of DBU, and the suspension was heated at 95°C for 22 hrs. The resulting orange solution was then evaporated to dryness, water was added to the residue, and the orange solid was collected and washed with methanol to give after drying 50 mg (60%) of yellowish solid, m.p. 240.3-242.3°C. ¹H NMR (TFA) : 9.8 (s, 1H), 9.14 (s, 1H), 8.77 (s, 1H), 5.78-5.55 (m, 1H), 4.76-4.12 (m, 5H), 2.9-2.65 (m, 2H), 1.65-1.2 (m, 4H).
Anal. calcd. for C₁₉H₁₈F₂N₆O₃; C, 54.81; H, 4.36; N, 20.17; Found; C, 54.52; H, 4.44; N, 20.00.

### Example 29

### 1-Cyclopropyl-6,8-difluoro-7-[3-(1,2,4-triazol-1-yl)pyrrolidin-1-yl]-1,4-dihydro-4-oxo-quinoline-3-carboxylic acid

To a solution of 1-cyclopropyl-6,7,8-trifluoro-1,4-dihydro-4-oxoquinoline-3-carboxylic acid (70.75 mg, 0.25 mmole) and 3-(1,2,4-triazol-1-yl)pyrrolidin-1-yl hydrochloride (100 mg) in 3 ml of dry pyridine under N₂ was added 152 mg (1 mmole) of DBU and the solution was heated at 95°C overnight. The orange solution was then concentrated under reduced pressure and water was added to the residue and the solid was collected and dried to yield 50 mg (50%) of a gray solid, m.p. 219.7-220.7°C. ¹H NMR (TFA) : 9.8 (s, 1H), 9.29 (s, 1H), 8.78 (s, 1H), 8.11 (d, J=12.5, 1H), 5.85-5.58 (m, 1H), 4.9-4.2 (m, 5H), 3.00-2.7 (m, 2H), 1.75-1.3 (m, 4H).
Anal. calcd. for C₁₉H₁₇F₂N₅O₃; C, 56.86; H, 4.27; N, 17.45. Found; C, 56.80; H, 4.41; N, 17.48.

### Example 30

### 1-Cyclopropyl-6,8-difluoro-7-[3-(1,2,4-triazol-1-yl)pyrrolidin-1-yl]-1,4-dihdyro-4-oxo-quinoline-3-carboxylic acid methanesulfate

1-Cyclopropyl-6,8-difluoro-7-[3-(1,2,4-triazol-1-yl)pyrrolidin-1-yl]-1,4-dihydro-4-oxo-quinoline-3-carboxylic acid (20 mg, 0.05 mmole) was dissolved in 2 ml of chloroform and to this was added 4 ml of methanesulfonic acid in chloroform. The suspension formed was stirred at room temperature for five hrs. The solid was collected to yield 12 mg of white solid after drying very well in the oven, m.p. 208.5-210°C. ¹H NMR (CD₃OD) : 9.54 (s, 1H), 8.78 (s, 1H), 8.64 (s, 1H), 7.81 (dd, 13.8, 2.3 Hz, 1H), 5.47-5.3 (m, 1H, 4.5-3.8 (m, 5H), 2.88-2.5 (m, 5H, singlet at 2.7), 1.4-1.1 (m, 4H).
Anal. calcd. for C₂₀H₂₁F₂N₅O₆S·H₂O; C, 46.60; H, 4.50; N, 13.59. Found; C, 46.75; H, 4.38; N, 13.51.

### Example 31

### 1-Cyclopropyl-6-fluoro-7-[3S-(1,2,3-triazol-1-yl)pyrrolidin-1-yl]-8-methoxy-1,4-dihydro-4-oxo-quinoline-3-carboxylic acid

To 50 mg (0.15 mmole) of ethyl 6,7-difluoro-1-cyclopropyl-8-methoxy-1,4-dihydro-4-oxo-quinoline-3-carboxylate was added 1ml of fluoroboric acid (50% in water) and the mixture was heated at 90-100°C for 3 hrs. The solution was then poured into water and solid collected (60 mg). The white solid was dissolved in 1 ml of DMSO and to this solution was added 52 mg (0.3 mmol) of 3S-(1,2,3-triazol-1-yl)pyrrolidine hyrochloride and 46 mg (0.3 mmol) of DBU. The mixture was then heated at 90°C for 42 hrs. The reaction mixture was cooled to room temperature and water was added and solid collected. This solid was dissolved in 8 ml of 80% methanol and 0.25 ml of triethylamine was added and refluxed for 4 hrs. The solution was cooled and the few particles were filtered. The supernatant was evaporated to dryness, and to the ethanol was added to the residue, the solid collected, washed with ether and dried to yield 10 mg of the desired product, m.p. 195-197°C. ¹H NMR (TFA) : 9.34 (s, 1H), 8.68 (s, 1H) 8.57 (d, 1H),8.09 (d, 13.2 Hz, 1H), 5.95-5.8 (m, 1H), 4.85-4.3 (m, 4H), 4.2-4.0 (m, 1H), 3.79 (s, 3H), 3.1-2.7 (m, 2H), 1.7-1.1 (m, 4H).

### Example 32

### 1-Cyclopropyl-6-fluoro-7-[3-(1,2,3-triazol-1-yl)pyrrolidin-1-yl]-8-methoxy-1,4-dihydro-4-oxo-quinoline-3-carboxylic acid

Ethyl 6,7-difluoro-1-cyclopropyl-8-methoxy-1,4-dihydro-4-oxo-quinoline-3-carboxylate was complexed with fluoroboric acid according to the procedure described in Example 31, and then reacted with 3-(1,2,3-triazol-1-yl)pyrrolidine under the same reaction conditions, followed by hydrolysis to yield the desired product, m.p. 200-201°C. ¹H NMR (TFA): 9.34 (s, 1H), 8.69 (d, 1H), 8.58 (d, 1H), 8.09 (d, 13.3 Hz, 1H), 5.93-5.8 (m, 1H), 4.83-4.34 (m, 4H), 4.2-4.0 (m, 2H), 3.79 (s,3H), 3.05-2.75 (m, 2H), 1.7-1.1 (m, 4H). Anal. calcd, for C₂₀H₂₀FN₅O₄ 1/2 H₂O; C, 56.87; H, 5.01; N, 16.58; Found; C, 56.86; H, 4.68; N, 16.24

### Example 33

### 1-Cyclopropyl-6-fluoro-7-[3-(4-methyl-1,2,3-triazol-1-yl)pyrrolidin-1-yl[-8-methoxy-1,4-dihydro-4-oxo-quinoline-3-carboxylic acid

The borane complex prepared according to the Example 31 is reacted with 3-(4-methyl-1,2,3-triazol-1-yl)pyrrolidine and the resulting conjugated product was subjected to the same hydrolysis procedure to yield 10 mg of the desired product, m.p. 174-176°C. ¹H NMR (TFA): 9.34 (s, 1H), 8.39 (s, 1H), 8.08 (d, 13.4 Hz, 1H), 5.84-5.16 (m, 1H), 4.78-4.18 (m, 4H), 4.2-4.0 (m, 1H), 3.78 (s, 3H), 3.08-2.7 (m, 2H), 2.67 (s, 3H), 1.75-1.05 (m, 4H). Anal. calcd. for C₂₁H₂₂FN₅O₄, 1 1/2 H₂O; C, 55.50; H, 5.54; N, 15.41. Found; C, 55.73; H, 4.95; N, 14.63.

### Example 34

### 1-Cyclopropyl-6-fluoro-7-[3-(1,2,4-triazol-1-yl)pyrrolidin-1-yl]-8-methoxy-1,4-dihydro-4-oxo-quinoline-3-carboxylic acid

The borane complex of ethyl 6,7-difluoro-1-cyclopropyl-8-methoxy-1,4-dihydro-4-oxo-quinoline-3-carboxylate (50 mg) was formed according to the procedure described for Example 31, and reacted in the same manner with 3-(1,2,4-triazol-1-yl)pyrrolidine hydrochloride. The product from this reaction was then hydrolyzed using triethylamine as described in example 31 to yield the desired product, m.p.221-222°C. ¹H NMR (TFA) : 9.82 (s, 1H) , 9.34 (s, 1H), 8.79 (s, 1H), 8.08 (d, 13.3 Hz, 1H), 5.85-5.64 (m, 1H), 4.8-4.3 (m, 4H), 4.25-4.0 (m, 1H), 3.81 (s, 3H), 3.0-2.7 (m, 2H), 1.7-1.1 (m, 4H).

### Example 35

### N,N,N-Trimethyl-N-(2-hydroxyethyl) ammonium 1- cyclopropyl-6,8-difluoro-7-[3-(4-methyl-1,2,3-triazol-1-yl)pyrrolidin-1-yl]-1,4-dihydro-4-oxo-quinoline-3-carboxylate

To a suspension of 1-cyclopropyl-6,8-difluoro-7-[3-(4-methyl-1,2,3-triazol-1-yl)pyrrolidin-1-yl]-1,4-dihydro-4- oxo-quinoline-3-carboxylic acid (415.4 mg, 1 mmole) in 2.5 ml of methanol and 0.8 ml water at room temperature was added slowly 0.31 ml of 45% N,N,N-trimethyl-2-hydroxyethyl-ammonium hydroxide in methanol (1 mmole). The solution was stirred at room temperature for an additional hour. This was filtered through the small amount of cotton and the supernatant was evaporated under reduced pressure at 30°C. The residue was crystallized from acetone-methanol to yield after drying 420 mg of off-white solid, m.p. 188.7-190.7°C. ¹H NMR (D₂O) : 8.44 (s, 1H), 7.84 (bs, 1H), 7.62 (bd, 13.9Hz, 1H), 5.43-5.22 (m, 1H), 4.35-3.62 (m, 7H), 3.52 (t, 2H), 3.21 (s, 9H), 2.73-2.16 (m, 2H) 2.29 (s, 3H), 1.74-0.9 (m, 4H). Anal. calcd. for C₂₅H₃₂F₂N₆O₄·1/2 H₂O: C, 56.98; H, 6.31; N, 15.94. Found: C, 56.76; H, 6.26; N, 15.43.

### EXAMPLE 36

### N-(2-Hydroxyethyl)ammonium-1-cyclopropyl-6,8-difluoro-7-[3-(4-methyl-1,2,3-triazol-1-yl)pyrrolidin-1-yl]-1,4-dihydro-4-oxo-quinoline-3-carboxylate

A suspension of 20 mg (0.05 mmole) of 1-cyclopropyl-6,8-difluoro-7-[3-(4-methyl-1,2,3-triazol-1-yl )pyrrolidin-1-yl]-1,4-dihydro-4-oxoquinoline-3-carboxylic acid in 2 ml of ethanol was reacted with 3.054 mg (0.05 mole) of the ethanolamine at room temperature for 2 hrs. Additional equivalent of ethanolamine was added and the reaction mixture was stirred overnight. The solid was then collected, m.p. 215.1-216.8°C. ¹H NMR (D₂O) : 8.46 (s, 1H), 7.86 (bs, 1H), 7.68 (bd, 14Hz, 1H), 5.46-5.25 (m, 1H), 4.4-3.7 (m, 7H), 3.17-3.07 (m, 2H), 2.76-2.2 (m, 5H, singlet at 2.31), 1.3-1. (m, 4H). Anal. calcd. for C₂₂H₂₆F₂N₆O₄ · H₂O: C, 53.44; H, 5.71; N, 16.99. Found: C, 53.68; H, 5.93; N, 16.48.

### EXAMPLE 37

### N,N,N-Trimethyl-N-(2-hydroxyethyl)ammonium-1-cyclopropyl-6,8-difluoro-7-[3-(1,2,4-triazol-1-yl)pyrrolidin-1-yl]-1,4-dihydro-4-oxo-quinoline-3-carboxylate To a suspension of 100.25mg (0.25 mmole) of 1-cyclopropyl-6,8-difluoro-7-[3-(1,2,4-triazol-1-yl)pyrrolidin-1-yl] - 1,4-dihydro-4-oxo-quinoline-3-carboxylic acid in 0.625 ml of methanol and 0.2 ml water was added at room temperature slowly 0.075 ml of 45% choline in methanol. The solution was stirred at room temperature for an additional hour and after being through a cotton filter, the supernatant was evaporated to dryness under reduced pressure (30°C). The residue was crystallized from acetone-methanol and dried to give 85 mg of off white solid, mp. 193-194.2°C. ¹H NMR (CD₃OD) : 8.61 (s, 1H), 8.42 (bs, 1H) 8.01 (s,1H), 7.76 (dd, 14,1.8Hz,1H), 5.3-5.15 (m,1H), 4.3-3.75 (m, 7H), 3.5-3.44 (m, 2H), 3.21 (s, 9H), 2.62-2.45 (m, 2H), 1.22-1.02 (m, 4H). Anal. calcd. for C₂₄H₃₀F₂N₆O₄ H₂O; C, 55.16; H, 6.13; N, 16.07. Found; C, 54.46; H, 5.91; N, 15.82.

### Example 38

### (-)-9-Fluoro-3(S)-methyl-10-[3-(1,2,3-triazol-1-yl)pyrrolidin-1-yl]-7-oxo-2,3-dihydro-7H-pyrido[1,2,3-de]-1,4-benzoxazine-6-carboxylic acid

To a suspension of 76 mg (0.27 mmole) of (-)-9,10-difluoro-3(S)-methyl-7-oxo-2,3-dihydro-7H-pyrido[1,2,3-de]-1,4-benzoxazine-6-carboxylic acid and 118 mg (0.675 mmole) of 3-(1,2,3-triazol-1-yl)pyrrolidine hydrochloride in 10 ml of dry acetonitrile was added 121 mg (0.81 mmole)of DBU. The reddish brown solution was refluxed for 31 hrs. The solvent was then removed under reduced pressure. To the residue water was added and extracted with chloroform. The organic layer was then evaporated to dryness, and to the residue water was added and solid collected to yield 10 mg of the desired product, m.p. 225-229°C. ¹H NMR (TFA) : 9.17 (s, 1H), 8.67 (s, 1H), 8.57 (s, 1H), 7.99 (d, 13.3 Hz, 1H), 5.9-5.18 (m, 1H), 5.22-4.97 (m, 7H), 3.13-2.63 (m, 2H), 1.82 (s, 3H), 1.79 (s, 3H).
Anal. calcd for C₁₉H₁₈N₅O₄F 1/2 H₂O; C, 55.86; H,4.69; N, 17.16. Found; C, 55.93; H, 4.59; N, 16.37.

### Example 39

### (-)-9-Fluoro-3(S)-methyl-10-[3-(4-methyl-1,2,3-triazol-1-yl]pyrrolidin-1-yl]-7-oxo-2,3-dihydro-7H-pyrido[1,2,3-de]-1,4-benzoxazine-6-carboxylic acid

A solution of 70.25 mg (0.25 mmole) of (-)-9,10-difluoro-3(S)-methyl-7-oxo-2,3-dihydro-7H-pyrido[1,2,3-de]-1,4-benzoxazine-6-carboxylic acid and 141 mg (0.75 mmole) of 3-(4-methyl-1,2,3-triazol-1-yl)pyrrolidine hydrochloride in 3 ml of acetonitrile in the presence of 152.24 mg (1 mmole) of DBU was refluxed for 3 days under nitrogen atmosphere. The suspension was then evaporated and water was added to the residue and solid collected and washed with methanol to yield after drying 50 mg of the desired product, m.p. 241-242°C. ¹H NMR (TFA) : 9.16 (s, 1H), 8.37 (s, 1H), 7.99 (d, 13.3 Hz, 1H), 5.75-5.6 (m, 1H), 5.15-4.1 (m, 7H), 3-2.6 (m, 5H, singlet at 2.67), 1.8 (d, 6.8 Hz, 3H). Anal. calcd. for C₂₀H₂₀FN₅O₄ · 1/2 H₂O; C, 56.87; H, 5.01; N, 16.58. Found; C, 57.30; H, 4.77; N, 16.75.

### Example 40

### (-)-9-Fluoro-3-(S)-methyl-100[3-(1,2,4-triazol-1-yl)pyrrolidin-1-yl]-7-oxo-2,3-dihydro-7H-pyrido[1,2,3-de]1,4-benzoxazine-6-carboxylic acid

A suspension of 70.25 mg (0.25 mmole) of (-)-9,10-difluoro-3(S)-methyl-7-oxo-2,3-dihydro-7H-pyrido[1,2,3-de]-1,4-benzoxazine-6-carboxylic acid and 87 mg (0.5 mmole) of 3-(1,2,4-triazol-1-yl)pyrrolidine hydrochloride in 3 ml of acetonitrile in the presence of 76 mg (0.5 mmole) DBU was stirred under nitrogen at reflux condition overnight. The yellow solution was then evaporated to dryness and to the residue water was added and solid collected. The off-white solid was dissolved in chloroform and the small amounts of the suspension were filtered off. The supernatant was evaporated and to the residue water was added and solid collected (60 mg), m.p. 229-231°C. ¹H NMR (CDCl₃) : 8.59 (s, 1H) , 8.26 (s, 1H), 7.98 (s, 1H), 7.7 (d, 13.5 Hz, 1H), 5.17-5.00 (m, 1H), 4.6-3.73 (m, 7H), 2.67-2.4 (m, 2H), 1.61 (d, 6.7 Hz, 3H).

### Example 41

### 5-Amino-1-cyclopropyl-6,8-difluoro-7-(3-(4-amino-1,2,3-triazol-1-yl)-pyrrolidin-1-yl)-1,4-dihydro-4-oxo-quinoline-3-carboxylic acid

A mixture of 5-amino-1-cyclopropyl-6,7,8-trifluoro-1,4-dihydro-4-oxo-quinoline-3-carboxylic acid (100 mg, 0.67 mmol), 3-(4-amino-1,2,3-triazol-1-yl)pyrrolidine dihydrochloride (185 mg, 0.83 mmol) and diazabicycloundecane (127 mg, 0.83 mmol) in pyridine (3 ml) was heated at 110°C for 24 hrs. Reaction mixture was concentrated to dryness under vacuum and the solid residue was dissolved in water, extracted with methylene chloride, dried over sodium sulfate and concentrated to dryness. The solid residue thus obtained was crystallized from chloroform to brown solid. Yield 15 mg (10.4%) m.p. 210-213°C. ¹H NMR (TFA) : 1.22-1.58 (m, 4H), 2.70-2.90 ((m, 2H), 4.06-4.54 (m, 5H), 5.48-5.60 (m, 1H), 7.79 (s, 1H), 9.14 (s, 1H) ppm.

### Example 42

### 1-Cyclopropyl-6,8-difluoro-7-(3-(4-amino-1,2,3-triazol-1-yl)-pyrrolidin-1-yl)-1,4-dihydro-4-oxo-quinoline-3-carboxylic acid

A mixture of 1-cyclopropyl-6,7,8-trifluoro quinolone-3-carboxylic acid (75 mg, 0.26 mmol), 3-(4-amino-1,2,3-triazol-1-yl)pyrrolidine (145 mg, 0.65 mmol) and diazabicycloundecane (198 mg, 1.30 mmol) in acetonitrile (5 ml) heated for 3 days at 110°C. Separated light brown solid was filtered, washed with water and then crystallized from methanol to 55 mg (50.9%) of title compound. m.p. 221°C, NMR (TFA) : 1.38-1.72 (m, 4H), 2.66-2.98 (m, 2H), 4.10-4.80 (m, 5H), 5.42-5.64 (m, 1H), 7.7-7.9 (m, 1H), 9.28 (s, 1H).

### Example 43

### 1-Cyclopropyl-6,8-difluoro-7-(3-(5-carboxy-1,2,3-triazol-1-yl)-pyrrolidin-1-yl)-1,4-dihydro-4-oxo-quinoline-3-carboxylic acid

A mixture of 5-amino-1-cyclopropyl 6,7,8-trifluoro-1,4-dihydro-4-oxo-quinoline-3-carboxylic acid (100 mg, 0.33 mmol), 3-(5-ethoxy-carbonyl-1,2,3-triazol-1-yl)-pyrrolidine hydrochloride (178 mg, 0.83 mmol) and diazabicycloundecane (127 mg, 0.83 mmol) was heated at 110°C for 24 hrs., concentrated, diluted with water and the separated solid was washed with water and acetronitrile. The solid was dried in air and redissolved in methanol (5 ml) and to it NaOH (20 mg) in water (5 ml) was added. The solution was heated to 90°C for 6 hrs, cooled and pH lowered using 1N HCl to 4. The separated solid was filtered, recrystallized from methanol/ether, yield 20 mg (13.98%), m.p. 214°C, NMR : 1.30-1.62 (m, 4H), 2.70-3.0 (m, 2H), 3.74-4.80 (m, 5H), 5.60-5.70 (m, 1H), 8.86 (s, 1H), 9.14 (s, 1H).

### Example 44

### 1-cyclopropyl-6,8-difluoro-7-[3-(5-aminomethyl-(1,2,3-triazol-1-yl)-pyrrolidin-1-yl]-1,4-dihydro-4-oxo-quinoline-3-carboxylic acid

A mixture of 1-cyclopropyl-6,7,8-trifluoro-1,4-dihydro-4-oxo-quinoline (200 mg, 0.706 mmol) and 3-(5-aminomethyl-1,2,3-triazol-1-yl)-pyrrolidine dihydrochloride (585 mg, 2.46 mmol) and diazabicycloundecane (325 mg, 2.48 mmol)in pyridine (8 ml) was heated at 110°C for 20 hrs, concentrated and the residue washed repeatedly with acetronitrile. The solid thus obtained was redissolved in chloroform and then washed with water, brine and dried using anhyd. sodium sulfate. Evaporation of chloroform yielded 120 mg (40.33%) of desired product, m.p. 138°C. (TFA) : 1.30-1.70 (m, 4H), 2.72-3.05 (m, 2H), 4.20-4.67 (m, 5H), 4.73-4.95 (m, 1H), 5.15 (s, 2H), 5.93-6.17 (m, 1H), 8.12 (d, 1H), 8.88-9.30 (s, 1H).

### Example 45

### 1-cyclopropyl-6,8-difluoro-7-[3-(4-carboxyl-1,2,3 -triazol-1-yl)pyrrolidin-1-yl]-1,4-dihydro-4-oxo-quinoline-3- carboxylic acid

A mixture of 1-cyclopropyl-6,7,8-trifluoro-1,4-dihydro-4-oxo-quinoline-3-carboxylic acid (140 mg, 0.49 mmol), 3-(4 carboxy-1,2,3-triazol-1-yl)pyrrolidine hydrochloride (269 mg, 1.23 mmol) and diazabicycloundecane (187 mg, 1.23 mmol) in acetonitrile 5 ml was heated at 80-90°C. for four days. The reaction mixture was concentrated diluted with water (10 ml) and pH brought down to 4.0 using 1M HCl. The separated solid was filtered and washed with water and crystallized from MeOH/ether to yellow solid. Yield 29 mg (13.2%), m.p. 222-225°C. ¹H NMR (CDCl₃ + CD₃OD) : 1.11-1.42 (m, 4H), 3.88-4.30 (m, 4H), 4.40-4.48 (m, 1H), 5.35-5.55 (m, 1H), 7.8 (d, 1H), 8.41 (s, 1H), 8.79 (s, 1H).

### Example 46

### 1-cyclopropyl-6,8-difluoro-7-[3-(4-phenyl-1,2,3-triazol-1-yl)-pyrrolidin-1-yl]-1,4-dihydro-4-oxo-quinoline-3-carboxylic acid

A mixture of 1-cyclopropyl-6,7,8-trifluoro-1,4-dihydro-4-oxo-quinoline-3-carboxylic acid (150 mg, 0.53 mmol), 3-(4 phenyl-1,2,3-triazol-1-yl) pyrrolidine hydrochloride (252 mg, 1.05 mmol) and diazabicycloundecane (160 mg, 1.05 mmol) in pyridine (5ml) was heated at 110°C for 20 hrs, concentrated, and diluted with water. The separated solid was filtered, washed with water and crystallised from methanol. Yield (55 mg, 22.3%) m.p. 241.5°C. ¹H NMR (TFA) : 1.34-1.73 (m, 4H), 2.82-3.09(m, 2H), 4.20-4.95 (m, 5H), 5.73-5.92 (m, 1H), 7.5-7.92 (m, 4H), 8.15 (d, 1H), 8.80 (s, 1H), 9.30 (s, 1H).

### Example 47

### 5-amino-1-cyclopropyl-6,8-difluoro-7-[3-(4-phenyl-1,2,3-triazol-1-yl)-pyrrolidin-1-yl]-1,4-dihydro-4-oxo-quinoline-3-carboxylic acid

A mixture of 5-amino-1-cyclopropyl-6,7,8-trifluoro-1,4-dihydro-4-oxo-quinoline-3-carboxylic acid (100mg, 0.33mmol), 3-(4-phenyl-1,2,3-triazol-1-yl)pyrrolidine hydrochloride (160mg, 0.42mmol) and diazabicycloundecane (102mg, 0.67mmol) in pyridine (5 ml) was heated at 90°C for 20 hrs. The reaction mixture was concentrated and the residue was diluted with water. The separated solid was filtered, washed with water, methanol and then with CHCl₃ followed by acetonitrile to get 16 mg (10%) of title compound. m.p. 265°C. ¹H NMR (TFA) : 0.92-1.43 (m, 4H), 2.51-2.80 (m, 2H), 3.57-4.59 (m, 5H), 5.43-5.62 (m, 1H), 7.44 (dd, 5H), 8.50 (s, 1H), 8.84 (s, 1H).

### Example 48

### 1-cyclopropyl-6,8-difluoro-5-methyl-7-[3(4-phenyl-1,2,3-triazol-1-yl)-pyrrolidin-1-yl]-1,4-dihydro-4-oxo-quinoline-3-carboxylic acid

A mixture of 1-cyclopropyl-6,7,8-trifluoro-5-methyl-1,4-dihydro-4-oxo-quinoline-3-carboxylic acid (100 mg, 0.37 mmol), 3-(-4-phenyl-1,2,3-triazol-1-yl)-pyrrolidine hydrochloride (160 mg, 0.67 mmol) and diazabicycloundecane (102 mg, 0.67 mmol) in (5ml) pyridine was heated at 90°C for 24 hrs. The reaction mixture was concentrated and diluted with water. The separated solid was filtered and washed with water followed by ether and was crystallized from methanol. Yield 89 mg (55%), m.p. 221.4°C. ¹H NMR (TFA) : 1.20-1.70 (m, 4H), 2.76-3.80 (m, 2H), 4.20-4.96 (m, 5H), 5.74-5.90 (m, 1H), 7.52 (dd, 5H), 8.70 (s, 1H), 9.27 (s, 1H).

### Example 49

### 1-cyclopropyl-5,6,8-trifluoro-7-[3-(1,2,3-triazol-1-yl)-pyrrolidin-1-yl]-1,4-dihydro-4-oxo-quinoline-3-carboxylic acid

A mixture of ethyl-1-cyclopropyl-5,6,7,8-tetrafluoro-1,4-dihydro-4-oxo-quinoline-3-carboxylate (150 mg, 0.45 mmol), 3-(1, 2, 3-triazol-1-yl) -pyrrolidine hydrochloride (216 mg, 1.24 mmol) and diazabicycloundecane (189 mg, 1.24 mmol) in pyridine (5 ml) was heated at 100°C for 24 hrs. The reaction mixture was concentrated and water added to it. The separated solid was filtered, dried and crystallized from CHCl₃/EtOH mixture. The 50 mg of ester thus obtained was dissolved in methanol (10 ml) and aqueous NaOH (10 mg in 7 ml water) was added. The mixture was heated under reflux for 4 hrs. The methanol was evaporated and the remaining aqueous solution was acidified to pH 5, the separated white solid filtered, washed with water and dried in air. Yield 22 mg (11%), m.p. 250-253°C. ¹H NMR (TFA) : 1.25-1.70 (m, 4H), 2.75-3.10 (m, 2H), 4.18-4.95 (m, 5H), 5.72-5.95 (m, 1H), 8.56 (s, 1H), 8.70 (s, 1H), 9.27 (s, 1H).

### Example 50

### 1-Cyclopropyl-5-hydroxy-6,8-difluoro-7-[-3-(1,2,3-triazol-1-yl)-pyrrolidin-1-yl]-1,4-dihydro-4-oxo-quinoline-3-carboxylic acid

A mixture of ethyl-1-cyclopropyl-5-hydroxy-6,7,8-trifluoro-1,4-dihydro-4-oxo-quinoline-3-carboxylate (100 mg, 0.30 mmol), 3-(1,2,3-triazol-1-yl)-pyrrolidine hydrochloride (106.42 mg, 0.61 mmol) and diazabicycloundecane (97 mg, 0.63 mmol) in pyridine (3 ml) was heated at 100°C for 24 hrs. The reaction mixture was concentrated to dryness and the residue diluted with water. The separated yellow solid was filtered and purified on silica column using CHCl₃ as eluant. The purified ester (30 mg) was dissolved in methanol (20 ml) and to it aqueous NaOH (10 mg in 7 ml H₂O) was added. The solution was heated under reflux for 4 hrs. Methanol was evaporated by distillation and the aqueous solution was acidified using 1N HCl. The precipitated solid was collected by filtration, washed with water and dried at 40°C under vacuum. Yield 22 mg (17.25%), ¹H NMR (TFA) : 1.25-1.62 (m, 4H), 2.72-3.03 (m, 2H), 4.10-4.86 (m, 5H), 5.72-5.88 (m, 1H), 8.55 (s, 1H), 8.67 (s, 1H), 9.14 (s, 1H).

### Example 51

### 1-Cyclopropyl-6,8-difluoro-7-[3-(1,2,3,4-tetrazol-2-yl) pyrrolidin-1-yl]-1,4-dihydro-4-oxo-quinoline-3-carboxylic acid

A solution of 1-cyclopropyl-6,7,8-trifluoro-1,4-dihydro-4-oxo-quinoline-3-carboxylic acid (28.3 mg, 0.1 mmol), 3-(1,2,3,4-tetrazol-2-yl)pyrrolidine hydrochloride (35.1 mg, 0.2 mmol) and DBU (45.6 mg, 0.3 mmol) in acetonitrile (2 ml) was refluxed under nitrogen for 23 hrs. The solvent was then evaporated to dryness. The residue was diluted with water and extracted with chloroform. The organic extract was dried over Na₂SO₄, concentrated and the residue was crystallized from methanol-water.
Yield: 12 mg, m.p. 192-199°C, 1N NMR (CDCl₃) : 8.7 (s, 1H), 8.5 (s, 1H), 7.8 (dd. 1H), 5.6-5.4 (m, 1H), 4.46-3.76 (m, 5H), 2.9-2.45 (m, 2H), 1.35-1.02 (m, 4H).

### EXAMPLE 52

### 1-Cyclopropyl-6,8-fluoro-7-[3-(1,2,3,4-tetrazol-1-yl)pyrrolidin-1-yl]-1,4-dihydro-4-oxo-quinoline-3-carboxylic acid

A solution of 1-cyclopropyl-6,7,8-trifluoro-1,4-dihydro-4-oxo-quinoline-3-carboxylic acid (42.3 mg, 0.15 mmol), 3-(1,2,3,4-tetrazol-1-yl)pyrrolidine hydrochloride (53 mg, 0.3 mmol) and DBU (68 mg) in acetonitrile (5 ml) was refluxed under nitrogen and stirring for 23 hrs. The reaction mixture was concentrated and the residue was triturated with water. The separated solid was filtered, washed with acetonitrile and dried to yield 28 mg of the product. m.p. 268-270°C. ¹H NMR (TFA) : 9.65 (bs, 1H), 9.28 (s, 1H), 8.1 (d, 1H), 5.85-5.65 (m, 1H), 4.9-4.15 (m, 5H), 3.0-2.7 (m, 2H), 1.70-1.25 (m, 4H), Anal. calcd. for C₁₈H₁₆F₂N₆O₃ · 1/2 H₂O: C, 52.56; N, 4.17; N, 20.44; Found C, 52.91; H, 3.84; N, 20.01.

### Preparation of Intermediates

### Example A

### N-Benzyl-3-(1,2,3-triazol-1-yl)-pyrrolidine

A solution of N-benzyl-3-azido-pyrrolidine (1.5 g, 0.0074 mol) in acetone was taken in a steel bomb and cooled in dry ice/acetone bath. To this cold solution acetylene (2.5 mg, 0.096 mol) was added under N₂ atmosphere. The sealed steel bomb was then heated in an oil bath at 75°C for 20 hrs. The vessel was cooled again in dry ice/acetone bath and excess of acetylene released slowly, while the temperature rose to the room temperature (r.t.). The reaction mixture was concentrated to yellow-brown oil. Crude yield: 2.02 g. Crude compound was purified on silica gel column to obtain the title compound as a light yellow-brown oil. Yield : 1.02 g (60%). ¹H MNR (CDCL₃): 2.0-3.3 (m, 6H), 3.7 (S, 2H), 5.3 (m, 1H), 7.45 (S, 5H), 7.8 (S, 1H), 7.95 (S, 1H).

### Example B

### 3-(1,2,3-triazol-1-yl)-pyrrolidine hydrochloride

To a solution of N-benzyl-3-(1,2,3-triazol-1-yl)pyrrolidine (1 g, 0.0044 mol), in methanol (50 ml) was added 10% Pd/C (100 mg) and conc. HCl (1 ml). The suspension was hydrogenated at r.t. and 3.44. 10⁵ Pa (50 psi) pressure for 20 hrs. The Pd/c was removed by filtration and the solution was concentrated. The residue was recrystallized from methanol/ether to obtain white crystalline title compound. Yield: 680 mg (89.46%). ¹H NMR (D₂O) : 2.40 - 2.80 (m,2H), 3.58-3.66 (m,2H), 3.90-3.92 (d, 2H), 5.58-5.67 (m, 1H), 8.02 (d, 1H), 8.26 (d, 1h).

### Example C

### N-(tert-butoxycarbonyl)-3S-(1,2,3-triazol-1-yl)-pyrrolidine

A solution of N-(tert-butoxycarbonyl)-3S-azido pyrrolidine (9.1 g, .043 mmol,[α]²⁴ _{D} = +40°, MeoH) and acetylene (27 g/mmol) in dry acetone (100 ml) was heated at 75°C for 24 hrs in a pressure reaction vessel. After release of an excess of acetylene, the reaction mixture was concentrated to give 10.1 g of crude oily product. The crude product was purified over silica gel using ethylacetate-hexane (4:1) as eluant. Yield 9.0 g (88%); [α]²⁴ D = + 25° (MeOH) ¹H NMR (CDCL₃) : 1.53 (S, 9H) , 2.53 (m, 2H) , 3.80 (m, 4H), 5.26 (m, 1H), 7.70 (S, 1H), 7.80 (S, 1H).

### Example D

### N-(tert-butoxycarbonyl)-3R-(1,2,3-triazol 1-yl) -pyrrolidine

Prepared by the same procedure as described in Example C from reaction of N-(tert-butoxycarbonyl)-3R-azido pyrrolidine ([α]²⁴_{D} = 37°, MeOH) and acetylene. Yield 91%, [α]_{D}²⁴ =25° (MeOH), ¹H NMR (CDCl₃) : 1.50 (S, 9H), 2.50 (m, 2H), 3.80 (m, 4H), 5.26 (m, 1H), 7.63 (S, 1H), 7.76 (S, 1H).

### Example E

### 3S-(1,2,3-triazol-1-yl)-pyrrolidine hydrochloride

A solution of N-(tert-butoxycarbonyl)-3S-(1,2,3-triazol-1-yl)pyrrolidine (1.0 g, 4.2 mmol) in 20 ml methanol and 2.5 ml of conc. HCl was stirred at room temperature for 30 minutes. The reaction mixture was concentrated and the residue was crystallized from methanol : ether to give 800 mg of the title compound as hydrochloride. [α]²⁴_{D} = + 8° (MeOH), m.p. 189-92°C; ¹H NMR (CD₃OD) : 2.23 (m, 2H), 3.20 (t, 2H), 3.50 (d, 2H), 5.30 (m, 1H), 7.93 (S, 1H), 8.23 (S, 1H).

### Example F

### 3R-(1,2,3-Triazol-1-yl)pyrrolidine hydrochloride

Prepared by following the same procedure as described in example E from N-(tert. butoxycarbonyl)-3R-(1,2,3-triazol-1-yl)-pyrrolidine. [α]²⁴_{D} - 8° (MeOH), m.p. 190-193°C; ¹H NMR(CD₃OD) δ : 2.20 (m, 2H), 3.18 (t, 2H), 3.48 (d, 2H), 5.28 (m, 1H), 7.57 (S, 1H), 7.80 (S, 1H).

### Example G

### trans 3-Hydroxy-4-(1,2,3-triazol-1-yl)-1-N-(tert -butoxycarbonyl) pyrrolidine

A solution of trans 3-hydroxy-4-azido-1-N-(tert.-butoxycarbonyl) pyrrolidine (3.8 g, 17 mmol) in dry acetone (50 ml) was heated at 75-80° for 40 hrs. After releasing the excess of acetylene, the reaction mixture was concentrated and the residue was purified on silica gel using ethyl acetate as eluant. Yield 3.66 g (87%) m.p. 91-93°,¹H NMR(CDCl₃) δ : 1.46 (S, 9H), 3.25-4.25 (m, 5H), 4.83 (m, 1H), 7.63 (S, 1H), 7.70 (S, 1H).

### Example H

### trans 3-Hydroxy-4-(1,2,3-triazol-1-yl) pyrrolidine hydrochloride

Prepared by following the same procedure as described in example E from trans 3-hydroxy-4-(1,2,3-triazol-1-yl)-1-N-(tert-butoxycarbonyl) pyrrolidine. Yield 89%, m.p.170-173°C; ¹H NMR(CD₃OD) δ : 3.00-3.80 (m, 4H), 4.33 (m, 1H), 5.00 (m,1H), 7.66 (S, 1H), 7.96 (S, 1H).

### Example I

### trans 3-[(Methylsulfonyl)oxy]-4-(1,2,3-triazol-1-yl)- 1-N-(tert-butoxycarbonyl) pyrrolidine

Methane sulfonyl choride (2.84 g, 24.8 mmole) was added slowly to an ice cooled solution of trans 3-hydroxy-4-(1,2,3-triazol-1-yl)-1-N-(tert. butoxycarbonyl) pyrrolidine (3.16 g, 12.4 mmol) and triethylamine (4.36 ml, 31.3 mmol) in dry CH₂Cl₂ (10 ml). The reaction mixture was stirred under N₂ at room temperature for 22 hrs and washed with saturated NaHCO₃ and brine solution. The dichloromethane layer was dried over Na₂SO₄ and concentrated to give the title product. Yield 4.0 g (97%). ¹H NMR (CDCL₃) δ : 1.5 (S, 9H), 3.07 (s, 3H), 4.07 (m, 4H), 5.43 (m, 2H), 7.70 (S, 1H), 7.90 (S, 1H).

### Example J

### cis 3-Azido-4-(1,2,3-triazol-1-yl)-1-N-(tert-butoxycarbonyl)pyrrolidine

A mixture of compound of example I (4.0 g, 0.012 mol), NH₄Cl (0.86 g, 0.016 mol), and sodium azide (4.69 g, 0.072 mole) in a mixture of DMF (32 ml) and water (3.8 mol) was heated at 100°C for 6 hrs. The reaction mixture was diluted with water and extracted with ethylacetate. The ethyl acetate extract was dried over Na₂SO₄ and concentrated. The residue was purified over silica gel using ethyl acetate: hexane (2:1) as eluant. Yield 2.85 (85%). ¹H NMR (CDCl₃) : 1.48 (S, 9H), 3.90 (m, 4H), 4.45 (m, 1H), 5.36 (m, 1H), 7.70 (S, 1H), 7.80 (S, 1H).

### Example K

### cis 3-Amino-4-(1,2,3-triazol-1-yl)-1-N-(tert -butoxycarbonyl)-pyrrolidine

A solution of compound of example J (2.85 g) in methanol (75 ml) was hydrogenated under 3.44.10⁵Pa (50 psi) hydrogen pressure over 10% Pd/c (1.1 g) at room temperature for 18 hrs. The mixture was filtered through CELITE (a registered trade mark) and concentrated to give the title compound. Yield 2.6 g (86%) ¹H NMR (CDCl₃): 1.5 (S, 9H), 3.30 (m, 1H), 4.0 (m, 4H), 5.13 (m, 1H), 7.70 (S, 1H), 7.83 (S, 1H).

### Example L

### cis 3-Amino-4-(1,2,3-triazol-1-yl)pyrrolidine

A solution of compound of example K (1.0 g) in trifluoroacetic acid (4 ml) was stirred at room temperature under nitrogen for 10 minutes and concentrated. The residue was dissolved in methanol and treated with basic resin (ANGA-316) and filtered. The filtrate was concentrated and purified by column chromatography over neutral alumina using methanol/CHCl₃ mixture as solvent. Yield 670 mg. ¹H NMR (CD₃OD) : 2.80 (m, 1H), 3.20-3.80 (m, 5H), 5.10 (m, 1H), 7.75 (s, 1H), 8.0 (S, 1H).

### Example M

### N-Benzyl-3-(4,5-dimethyl-1,2,3-triazol-1-yl)pyrrolidine

To a solution of N-benzyl-3-azidopyrrolidine (2.02 g, 0.01 mole) in 30 ml of 98% ethanol was added 15 ml of butyne and heated in a sealed steel reaction vessel at 105-110°C for 24 hrs. Removal of the solvent under reduced pressure afforded 2.55 g of the crude product which contained 50% starting material. Chromatography over silica gel using hexane/ethyl acetate (1:1) as solvent yielded 0.88 g of the desired product. ¹H NMR (CDCl₃) : 7.36-7.2 (m, 5H), 4.95-4.8 (m, 1H), 3.7 (S, 2H), 3.2-3.1 (m, 1H), 2.93-2.76 (m, 3H), 2.48-2.3 (m, 2H), 2.24 (s, 3H), 2.21 (s, 3H).

### Example N

### 3-(4,5-Dimethyl-1,2,3-triazol-1-yl) pyrrolidine hydrochloride

To a solution of 0.86 g of N-benzyl-3-(4,5-dimethyl-1,2,3-triazol-1-yl)pyrrolidine in 50 ml of methanol and 2 ml of 1N hydrochloric acid was added 0.5 ml of concentrated hydrochloric acid followed by the addition of 10% Pd/c (.05 g). The suspension was then hydrogenated at 3.44 .10⁵ Pa (50 psi) pressure overnight. The Pd/C was removed by filtration through CELITE (a registered trade mark) and the supernatant was evaporated to dryness and crystallized from methanol-ether to yield 0.5 g of the desired product. ¹H NMR (D₂O) : 5.75-5.6 (m, 1H), 4.14-3.9 (m, 2H), 3.8-3.6 (m, 2H), 2.86-2.4 (m, 8H, two singlets at 2.49 and 2.4).

### Example O

### N-Benzyl-3-(4-methyl-1,2,3-triazol-1-yl)pyrrolidine N-Benzyl-3-(5-methyl-1,2,3-triazol-1-yl)pyrrolidine

A solution of 2.02 g (0.01 mole) of N-benzyl-3-azidopyrrolidine and 30 ml of 98% ethanol in a steel reaction vessel was cooled to -78°C and into this was bubbled 17 g of the propyne. The sealed reaction vessel was then heated at 110°C for 2.5 days. Removal of the solvent at reduced pressure resulted in 3 g of the crude product. Purification over silica gel (5% methanol ethyl acetate) afforded in the order of elution, 1.47 g of N-benzyl-3-(4-methyl-1,2,3-triazol-1-yl)pyrrolidine. ¹H NMR (CDCl₃) : 7.54 (s, 1H), 7.42-7.2 (m, 5H), 5.3-5.1 (m, 1H), 3.68 (s, 2H), 3.15-2.75 (m, 3H), 2.65-2.4 (m, 5H, singlet at 2.35) and 0.87 g of N-benzyl-3-(5-methyl-1,2,3-triazol-1-yl)pyrrolidine. ¹H NMR (CDCl₃) : 7.43 (s, 1H), 7.36-7.22 (m, 5H), 5.02-4.85 (m, 1H), 3.71 (s, 2H), 3.24-3.12 (s, 2H), 3.24-3.12 (m, 1H), 2.95-2.8 (m, 3H), 2.5-2.35 (m, 2H), 2.31 (s, 3H).

### EXAMPLE P

### 3-(4-Methyl-1,2,3-triazol-1-yl)pyrrolidine hydrochloride

Prepared using the same procedure described in Example N by hydrogenating 1 g of N-benzyl-3-(4-methyl-1,2,3-triazol-1-yl) pyrrolidine. The yellow viscous liquid obtained after evaporation was crystallized from methanol ether to yield 0.82 g of white solid. ¹H NMR (D₂O): 8.12 (S, 1H), 5.67-5.55 (m, 1H), 3.95 (d, 5.3 Hz, 2H), 3.67 (d, 6.26 Hz, 1H), 3.63 (d, 6.1 Hz, 1H). 2.85-2.45 (m, 2H), 2.42 (S, 3H).

### EXAMPLE Q

### 3-(5-Methyl-1,2,3-triazol-1-yl) pyrrolidine hydrochloride

Hydrogenation of 0.87 g of the N-benzyl-3-(5-methyl-1,2,3-triazol-1-yl) pyrrolidine according to the procedure described for Example N afforded 0.708 g of the desired product as a white salt after purification from methanol-ether. ¹H NMR (D₂O): 7.63 (S, 1H), 5.7-5.5 (m,1H), 4.25-3.6 (m, 4H), 2.87-2.37 (m, 5H, singlet at 2.51).

### Example R

### N-Benzyl-3-(1,2,4-triazol-1-yl)pyrrolidine

To a solution of 2.55 g (10 mmole) of N-benzyl-3-[(methylsulfonyl)oxy] pyrrolidine in 100 ml of dimethylformamide was added 3.21 g of potassium-1,2,4-triazolide and the reaction mixture was heated at 85-90°C overnight. To this, water (200 ml) was added and extracted with ethyl acetate. The combined organic layers were washed with water, dried (Na₂SO₄) and evaporated to yield the crude product which upon purification over silica gel using acetate as solvent yielded 1.32 g (15% methanol-ethyl acetate as solvent) of the title product. ¹H NMR (CDCl₃): 8.3 (s, 1H), 7.93 (s, 1H), 7.6-7.1 (M, 5H), 5.2-4.6 (M, 1H), 3.67 (s, 2H), 3.23-2 (m, 6H).

### Example S

### 3-(1,2,4-Triazol-1yl)pyrrolidine hydrochloride

The N-benzyl-3-(1,2,4-triazol-1-yl) pyrrolidine (1.3g) was hydrogenated at 3.44 10⁵ Pa (50 psi) for 3 days to yield after crystallization from methanol-ether 1.08 g of white solid. ¹H MNR (D₂O): 9.24 (s, 1H), 8.51 (s, 1H), 5.68-5.5 (m, 1H), 3.97-3.5 (m, 4H), 2.86-2.4 (m, 2H).

### Example T

### N-benzyl-3-(4-ethoxycarbonyl-1,2,3-triazol -1-yl)pyrrolidine and N-benzyl-3-(5-ethoxycarbonyl -1,2,3-triazol-1-yl)pyrrolidine

A mixture of N-benzyl-3-azido-pyrrolidine (5 g. 0.024 mmol) and ethylpropiolate (5 g, 0.051 mmol)in benzene (50 ml) heated under reflux for 48 hrs. Reaction solution was evaporated to dark brown oil, purified on silica gel column using ethylacetate, CHCl₃ and methanol to get N-benzyl-3-(5-ethoxycarbonyl-1,2,3-triazol-1-yl)pyrrolidine (1.5 g, 20.6%). NMR (CDCL₃): 1.37 (t, 3H), 2.42-2.58 (M, 2H), 2.71-3.01 (m, 3H), 3.20-3.32 (m, 1H), 3.73 (s, 2H), 4.35 (q, 2H), 5.72-5.88 (m, 1H), 7.2-7.4 (m, 5H), 8.12 (s, 1H). N-benzyl-3-(4-ethoxylcarbonyl-1,2,3-triazol-1-yl) pyrrolidine (5 g, 68.7%). NMR (CDCL₃): 1.40 (t, 3H), 1.8-3.20 (m, 6H), 3.70 (s, 2H), 4.40 (q, 2H), 5.06-5.48 (m, 1H), 7.33 (s, 5H), 8.43 (s, 1H).

### Example U

### N-benzyl-3-(4-carboxy-1,2,3-triazol-1-yl) pyrrolidine hydrochloride

A solution of N-benzyl-3-(4-ethoxycarbonyl-1,2,3-triazol-1-yl)-pyrrolidine (5 g) in 5N HCl (200 ml) was heated under reflux at 110°C for 16 hrs. The solution was evaporated under vacuum to dryness and the residue was crystallised from methanol/ether. Yield 3.6 g, 70.17%). NMR (D₂O): 2.11-2.72 (m, 2H), 3.30-3.90 (m, 4H), 4.27 (s, 2H), 5.18-5.48 (m, 1H), 7.30 (s, 5H), 8.30 (s, 1H)

### Example V

### 3-(4-carboxy-1,2,3-triazol-1-yl) pyrrolidine hydrochloride

To a solution of N-benzyl-3-(4-carboxy-1,2,3-triazol-1-yl)pyrrolidine hydrochloride (1 g, 0.0033 mol) in methanol (50 ml) was added 10% Pd/C (100 mg) and conc. HCl (0.5 ml). The suspension was hydrogenated at r.t. and 3.44. 10⁵Pa (50 psi) pressure for 20 hrs. The Pd/c was removed by filtration and the solution was concentrated. The residue was recrystallised from methanol/ether to obtain white crystalline title compound. Yield 650 mg (93.25%) MNR (D₂O) : 2.51-3.56 (m, 2H), 3.62-3.75 (m, 2H), 3.92-4.00 (m, 2H), 5.52-5.67

### Example W

### N-benzyl-3-(4-phenyl-1,2,3-triazol-1-yl)pyrrolidine and N-benzyl-3-(5-phenyl-1,2,3-triazol-1-yl)pyrrolidine

A solution of N-benzyl-3-azido pyrrolidine (3 g, 0.0148 mol) and phenyl-acetylene (3 g, 0.0292 mol) in benzene (30 ml) was heated under reflux for 48 hrs. The reaction mixture was concentrated to oil from which the title compounds were isolated by column chromatography using silica gel and a mixture of CHCl₃, hexane, MeOH (4:4:1) as eluant. N-benzyl-3-(5-phenyl-1,2,3-triazol-1-yl)pyrrolidine was obtained as oil. Yield 1 g (22.17%) ¹H NMR (CDCl₃) : 2.32-2.51 (m, 2H), 2.88-3.13 (m, 2H), 3.80 (s, 2H), 5.00 (p, 1H), 7.20-7.53 (m, 10H), 7.67 (s, 1N). N-benzyl-3-(4-phenyl-1,2,3-triazol-1-yl)-pyrrolidine, oil, yield 3.0g ¹H NMR (CDCl₃): 2.05-2.22 (m, 1H), 2.30-2.69 (m, 2H), 2.75-3.25 (m, 3H), 5.20-5.33 (m, 1H), 7.23-7.51 (m, 8H), 7.81-7.92 (m, 2H), 8.06 (s, 1H).

### Example X

### 3-(4-phenyl-1,2,3-triazol-1-yl)pyrrolidine hydrochloride

A suspension of N-benzyl-3-(4-phenyl-1,2,3-triazol)pyrrolidine (2.0 g), concentrated HCl (0.5 ml) and 10% Pd/C (200 mg) in methanol was hydrogenated following the procedure given in example 50. Yield 1.32 g ¹H NMR (D₂O): 2.35-2.72 (m, 2H), 3.57 (z, 2H), 3.84 (d, 2H), 5.35-5.45 (m, 1H), 7.35-7.65 (m, 5H), 8.23 (s, 1H).

### Example Y

### N-benzyl-3-(4-hydrazino carbonyl-1,2,3-triazol -1-yl)pyrrolidine

A suspension of N-benzyl-3-(4-ethoxycarbonyl-1,2,3-triazol-1-yl)pyrrolidine (5.6 g, 0.0186 mol) and NH₂NH₂H₂O (2.36 g, 0.067 mol) in water (1.6 ml) was heated to 120°C for 10 hrs. The reaction mixture was then cooled to r.t. and stirred with 15 ml of ether. The separated solid was filtered and washed with water. The white solid thus obtained was dried in oven at 40°C. Yield 3.8 g (71.29%), m.p. =122.6°C. ¹H NMR : 1.96-2.14 (m, 1H), 2.36-3.15 (m,5H), 3.7 (s, 2H), 4.05 (bs, 2H), 5.20-5.34 (m, 1H), 7.3 (s, 5H), 8.24 (s, 1H), 8.39 (s, 1H).

### Example Z

### N-Benzyl-3-(4-azidocarbonyl-1,2,3-triazol -1-yl)pyrrolidine

N-benzyl-3-(4-hydrazinocarbonyl-1,2,3-triazol-1-yl)pyrrolidine (3.6 g, 0.0125 mol) was dissolved in hot water (40 ml) and then cooled to 0°C. To the cooled suspension NaNO₂ (950 mg, 0.0137 mol in 3 ml of H₂O) was added dropwise and then glacial AcOH (7 ml) added to the reaction mixture was stirred at 0°C for 15 mm. Reaction mixture was then neutralized by the addition of saturated NaHCO₃. The separated white solid was filtered and washed with water, dried in vacuum oven. Yield 3.73 g (100%), m.p. 160°C. ¹H NMR (CDCl₃) : 1.95-2.15 (m, 1H), 2.37-2.83 (m, 3H), 2.94-3.22 (m, 2H), 3.70 (d, 2H), 5.75-5.87 (m, 1H), 7.30 (s, 5H), 8.47 (s, 1H).

### Example AA

### N-Benzyl-3-(4-t-butoxycarbonyl-amino-1,2,3-triazol -1-yl)pyrrolidine

A solution of N-benzyl-3-(4-azidocarbonyl-1,2,3-triazol-1-yl)pyrrolidine (1 g, 2.91 mmol) in t-butanol (15 ml) was refluxed for 15 hrs. The reaction mixture was evaporated to dryness. Pure title compound was obtained as oil by purification on silica gel column using CHCl₃/MeOH as eluant. Yield 700 mg (60.86%). ¹H NMR (CDCl₃) : 1.52 (s, 9H), 2.41-2.65 (m, 2H), 2.77-3.11 (m, 4H), 3.70 (s, 2H), 3.56-3.73 (m, 1H), 7.30 (s, 5H), 7.94 (s, 1H).

### Example BB

### N-Benzyl-3-(4-amino-1,2,3-triazol-1-yl) pyrrolidine dihydrochloride

A solution of N-benzyl-3-(4-t-butoxycarbonylamino-1,2,3-triazol-1-yl)pyrrolidine (100 mg, 0.291 mmol) and conc. HCl (0.5 ml) in methanol (5 ml) was stirred at r.t for 30 min. The solution was evaporated to dryness and the residue was crystallized from methanol/ether to obtain title compound as a solid. Yield: 90 mg (98.9%). NMR (D₂O) : 2.52-3.0 (m, 2H), 3.55-4.70 (m, 4H), 4.60 (s, 2H), 5.50-5.76 (m, 1H), 7.60 (s, 1H), 8.03 (s, 1H).

### Example CC

### 3-(4-amino-1,2,3-triazol-1-yl) pyrrolidine dihydrochloride

To a solution of N-benzyl-(4-t-butoxycarbonylamino-1,2,3-triazol-1-yl)-pyrrolidine-dihydrochloride (700 mg) in methanol 5% Pd/C (200 mg) was added. The suspension was hydrogenated at r.t. and 3.44.10⁵ P (50 psi) pressure over 30 hrs. The Pd/c was removed by filtration and the solution was concentrated. The residue was crystallized from methanol/ether to a white solid. Yield : 440 mg (95.8%). NMR (TFA) : 2.70-3.16 (m, 2H), 3.8-4.50 (m, 4H), 5.60-6.15 (m, 1H), 8.12 (s, 1H). The title compound was also obtained by hydrogenation of N-benzyl-(4-amino-1,2,3-triazol-1-yl)pyrrolidine hydrochloride in a yield of 92% following the procedure as given above.

### Example DD

### N-Benzyl-3-(1,2,3,4-tetrazol-1-yl)pyrrolidine and N-Benzyl-3-(1,2,3,4-tetrazol -2-yl)pyrrolidine

A suspension of N-benzyl-3-[(methylsulphonyl)oxy] pyrrolidine (2.2 g, 9.87 mmol) and sodium 1,2,3,4-tetrazolide (1.8 g, 19.6 mmol) in 50 ml of dimethylformamide was heated at 85-90°C overnight. The reaction mixture was worked up the same as described in Example I. The crude product was purified over silica gel using hexane, ether and ethyl acetate as eluant to afford in order of elution 0.413 g of N-benzyl-3-(1,2,3,4-tetrazol-2-yl)pyrrolidine as an oil. ¹H NMR (CDCl₃) : 8.51 (s, 1H), 7.37-7.17 (m, 5H), 5.51-5.35 (m, 1H), 3.8-3.65 (q, 2H), 3.3-2.4 (m, 6H); and .26 g of N-benzyl-3-(1,2,3,4-tetrazol-1-yl)pyrrolidine as an oil. H NMR (CDCl₃) : 8.88 (s, 1H), 7.4-7.2 (m, 5H), 5.35-5.2 (m, 1H), 3.83-3.6 (q, 2H), 3.2-2.3 (m, 5H), 2.15-2.00 (m, 1H).

### Example EE

### 3-(1,2,3,4-tetrazol-2-yl)pyrrolidine hydrochloride

The N-benzyl-3-(1,2,3,4-tetrazol-2-yl)pyrrolidine (0.4 g) was hydrogenated under similar conditions as described for Example J for 21 hrs to yield 280 mg of title product as hydrochloride salt after crystallization with methanol ether. ¹H NMR (D₂O) : 8.84 (s, 1H), 6.05-5.86 (m, 1H), 4.18-3.87 (m, 2H), 3.8-3.6 (m, 2H), 2.94-2.55 (m, 2H).

### Example FF

### 3-(1,2,3,4-tetrazol-1-yl)pyrrolidine hydrochloride

The N-benzyl-3-(1,2,3,4-tetrazol-1-yl)pyrrolidine (0.214 g) was hydrogenated under similar conditions as described for Example J for 21 hrs to yield 80 mg of title product as hydrochloride salt. ¹H NMR (D₂O) : 9.31 (s, 1H), 5.82-5.63 (m, 1H), 4.02-3.85 (m, 2H), 3.8-3.56 (m, 2H), 2.88-2.44 (m, 4H).

The structures of the intermediates and of compounds of the invention were established by the modes of synthesis and by extensive high field nuclear magnetic resonance spectral techniques.

The compounds of the invention display antibacterial activity when tested by the broth microdilution method as described in the NCCLS publications M7-A, M11-A and M17-P of the year 1985. For aerobic microorganisms, a cation supplemented Mueller Hinton Broth (BBL) was used whereas the Anaerobe Broth MIC (Difco) was used for anaerobic microorganisms. An agar Dilution method was employed for certain aerobic microorganisms using Mueller Hinton II Agar (BBL) and a Cathra multipoint inoculating device which delivered 10⁴ cfu/spot of the inoculum on the agar surface. MICs were read after 16-18 hours and 48 hours of incubation in case of microbes growing aerobically and anaerobically respectively.

By use of the above method, the following minimum inhibitory concentration values (MICs in µg/ml) were obtained: Table 1, against microbes growing aerobically; Table 2, against microbes growing anaerobically; Table 3, against methicillin resistant Staphylococcus aureus and quinoline resistant-methicillin resistant Staphylococcus aureus.

**TABLE 1**

| In vitro Antimicrobial Activity Against Aerobes MIC (µg/ml) | |
|---|---|
| Example No. | Staphylococcus aureus S-127 |
| 2 | ≤.06 |
| 4 | ≤.06 |
| 5 | ≤.03 |
| 6 | ≤.03 |
| 7 | .008 |
| 12 | .008 |
| 13 | .008 |
| 14 | ≤.015 |
| 15 | .015 |
| 16 | .015 |
| 17 | 1 |
| 18 | .5 |
| 19 | .5 |
| 20 | - |
| Sparfloxacin | .06 |
| Ciprofloxacin | .25 |

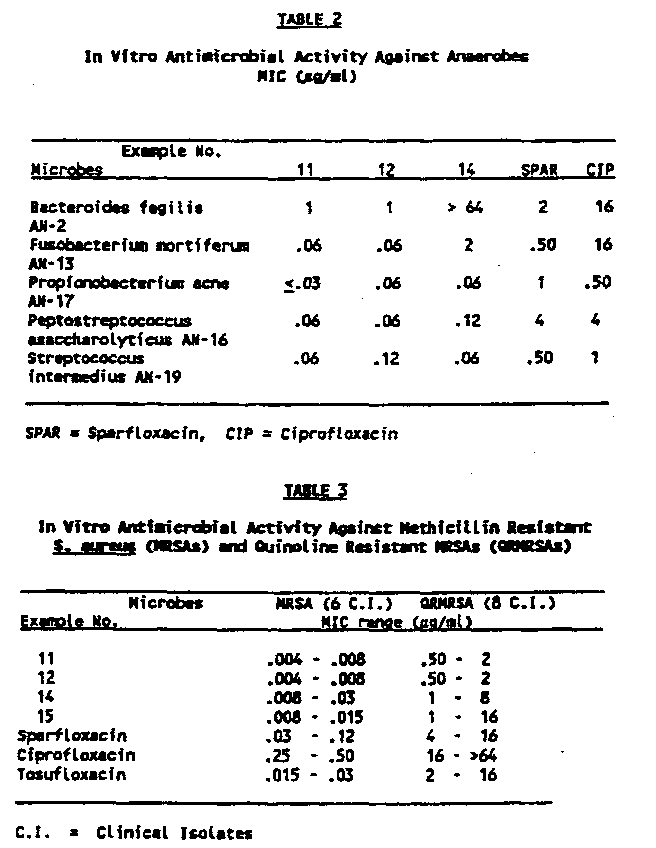

In another test procedure, the compounds of the present invention showed antibacterial activity when tested by the standard broth microdilution method as described in the NCCLS document M7-A2. Approved standard: Method for Dilution Antimicrobial Susceptability Tests for Bacteria that Grow Aerobically - Second Edition, 1990. The test organisms were obtained from ATCC and clinical laboratories. The 96-well microtitre plates containing 100 ml of serially diluted test compounds in Mueller Hinton Broth (MHB) (conc. range 64-0.03 µg/ml) were inoculated with 100 ml of bacterial suspension in MHB so that an inoculum size of 5 x 10⁵ cfu/ml was achieved. The plates were shaken gently and incubated at 35°C for 18 hr after which the minimum inhibitory concentrations (MIC, µg/ml) at which no visible microbial growth occurred were recorded.

### The abbreviations for the test organisms used Table 4 below are as follows:

- EC. (S- 63):: Eschericia coli
- Ecl. (S- 130):: Enterobacter cloacae
- Pa. (S- 67):: Pseudomonas aeruginosa
- Kp. (S- 80):: Klebsiella pneumoniae
- Pr. (S- 121):: Providencia rettgeri
- Sa. (S- 127):: Staphylococcus aureus

### ANTIMICROBIAL ACTIVITY (MIC, µG/ML)

### Microbroth Dilution (MHB) method; Inoculum, 5 x 10⁵ cfu/ml; Incubation 35 °C/18 hr

| Example No. | Ec S-63 | Ecl S-130 | Pa S-67 | Kp S-80 | Pr S-121 | Sa S-127 | Sa 127M |
|---|---|---|---|---|---|---|---|
| 21 | .06 | .25 | 2 | .25 | .12 | .015 | .015 |
| 22 | .25 | .25 | 4 | .5 | .25 | ≤.015 | .12 |
| 23 | .12 | .25 | 4 | .5 | .5 | ≤.015 | ≤.015 |
| 24 | .12 | .25 | 4 | .5 | .5 | ≤.015 | ≤.015 |
| 25 | .06 | .25 | 2 | .25 | .25 | ≤.015 | .03 |
| 26 | .25 | 2 | 8 | 1 | 1 | .03 | .06 |
| 27 | .25 | 1 | 4 | .5 | 2 | .125 | .25 |
| 28 | .12 | | | | | ≤.015 | |
| 29 | .12 | .25 | 4 | .5 | .25 | .03 | .06 |
| 31 | .12 | .5 | 2 | .25 | .5 | ≤.015 | ≤.015 |
| 32 | .12 | .5 | 4 | .25 | .12 | ≤.03 | ≤.03 |
| 33 | .12 | 1 | 8 | 1 | 2 | ≤.015 | ≤.015 |
| 34 | .5 | .5 | 8 | .5 | .5 | .5 | |
| 35 | .25 | .5 | 4 | .25 | .25 | ≤.015 | .03 |
| 37 | .5 | 2 | 8 | 1 | 1 | .06 | .25 |
| 38 | .25 | 1 | 8 | 1 | 1 | .12 | .5 |
| 39 | .25 | 1 | 8 | 1 | 1 | .03 | .12 |
| 40 | 1 | 1 | 8 | 1 | .5 | 1 | .5 |
| 39 | .25 | 1 | 8 | 1 | 1 | .03 | .12 |
| 40 | 1 | 1 | 8 | 1 | .5 | 1 | .5 |
| 41 | .12 | - | - | - | - | .03 | - |
| 42 | .5 | 1 | 4 | 1 | 1 | .25 | 1 |
| 43 | 16 | 32 | >32 | 32 | 32 | 8 | 8 |
| 44 | .5 | .25 | 8 | 1 | 1 | 25 | - |
| 45 | .25 | - | - | - | - | - | 1 |
| 46 | .25 | 1 | 32 | 1 | .015 | .015 | .015 |
| 47 | .25 | 16 | >32 | .5 | .015 | .015 | .015 |
| 48 | 1 | 4 | 16 | 4 | .015 | .015 | .015 |
| 49 | .5 | .50 | 8 | .5 | .12 | .25 | .25 |
| 50 | 1 | 2 | 16 | 1 | .06 | .25 | .25 |
| 51 | .06 | - | 2 | .12 | .12 | ≤.03 | ≤.03 |
| 52 | .06 | - | 2 | .25 | .12 | .06 | - |
| CPLX | ≤.008 | .03 | .25 | .015 | .015 | .25 | 8 |
| MFLX | .06 | .12 | 1 | .12 | 1 | 1 | 16 |
| MFLX- | .06 | .25 | 1 | .25 | .12 | 2 | 16 |
| CPLX = Ciprofloxacin NFLX = Norfloxacin NFLX-ch - Norfloxacin - choline salt | | | | | | | |

The compounds disclosed and described could be used in compositions to disinfect surfaces in environments where food is prepared.

## Claims

1. A 7-substituted-6-fluoro-1,4-dihydro-4-oxo-quinoline-3-carboxylic acid compound of the formula: 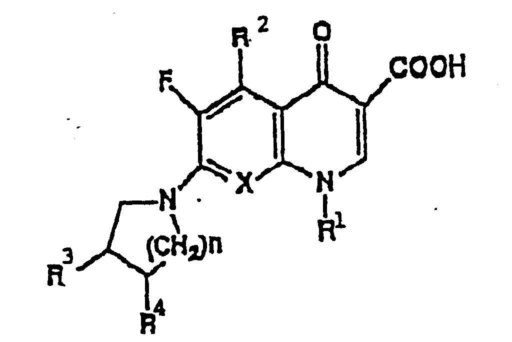 wherein R¹ is a C₃-C₆ cycloalkyl group or a phenyl group which may be substituted by methoxy or amino or by one or two halogen atoms; R² is hydrogen, a halogen atom, a C₁-C₄ alkyl group, a hydroxy group or an amino group;
R³ is hydrogen, hydroxy or amino;
R⁴ is a 1,2,3-triazol-1-yl group, a 1,2,4-triazol-1-yl group, a 1,2,3,4-tetrazol-1-yl or a 1,2,3,4-tetrazol-2-yl group, each of which may have 1 to 2 substituents selected from the group consisting of C₁-C₄ alkyl, COOH, CH₂NH₂, amino and phenyl groups; and X is N, CH, C-F or C-OCH₃; n is 1; or 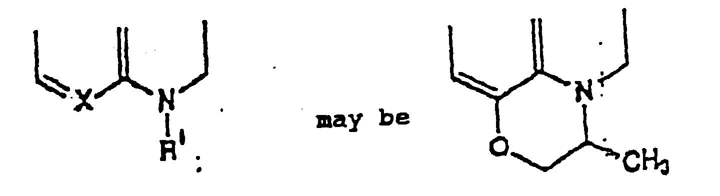 or a pharmaceutically acceptable salt thereof.

2. A compound according to claim 1, wherein R¹ is phenyl, 4-fluorophenyl, 2,4-difluorophenyl, 2,4-dimethoxyphenyl or 4-aminophenyl.

3. A compound according to claim 1, wherein R¹ is optionally substituted by chloro, fluoro, bromo, methoxy or amino.

4. A compound according to claim 1, wherein the compound has an asymmetric carbon atom on the pyrrolidine ring, and the compound is the R isomer, the S isomer or mixtures thereof.

5. A compound according to claim 1, wherein the compound has two asymmetric carbon atoms on the pyrrolidine ring, and the compound is a stereoisomer of the cis or trans configuration, or mixtures thereof.

6. A process for preparing a 7-substituted-6-fluoro-1, 4-dihydro-4-oxo-quinoline-3-carboxylic acid compound of the formula: 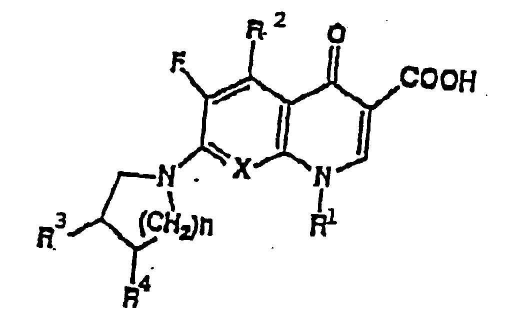 wherein R¹ is a C₃-C₆ cycloalkyl group or a phenyl group which may be substituted by methoxy or amino or by one or two halogen atoms; R² is hydrogen, a halogen atom, a C₁-C₄ alkyl group, a hydroxy group or an amino group;
R³ is hydrogen, hydroxy or amino;
R⁴ is a 1,2,3-triazol-1-yl group, a 1,2,4-triazol-1-yl group, a 1,2,3,4-tetrazol-1-yl or a 1,2,3,4-tetrazol-2-yl group, each of which may have 1 to 2 substituents selected from the group consisting of C₁-C₄ alkyl, COOH, CH₂NH₂, amino and phenyl groups; and
X is N, CH, C-F or C-OCH₃;
n is 1; or 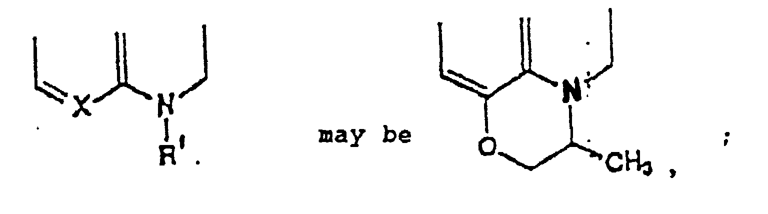 or a pharmaceutically acceptable salt thereof, said process comprising reacting a compound of the formula 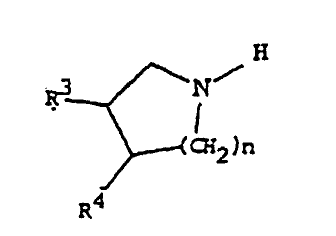 with a compound of the formula 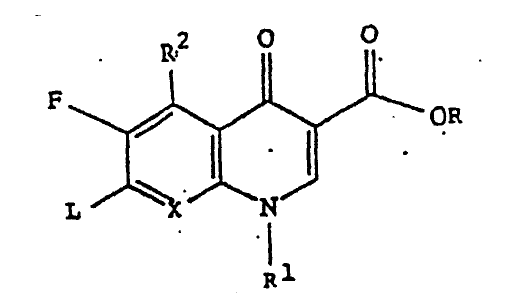 wherein R¹, R², R³, R⁴, n and X are as defined above; L is a leaving group selected from chlorine, fluorine and SO₂R₇, wherein R₇ is a C₁-C₄ alkyl group or an unsubstituted or substituted phenyl group.

7. A pharmaceutical composition suitable for treating bacterial infections comprising an antibacterial amount of a compound of claim 1 and a pharmaceutically acceptable carrier.

8. A method for disinfecting a surface to be disinfected which comprises treating the surface with a composition containing an antibacterial effective amount of a compound of claim 1.

9. Use of a compound according to claim 1 in the manufacture of a medicament for treatment of a human suffering from a bacterial infection.

## Patentansprüche

1. 7-Substituierte 6-Fluor-1,4-dihydro-4-oxochinolin-3-carbonsäure der Formel 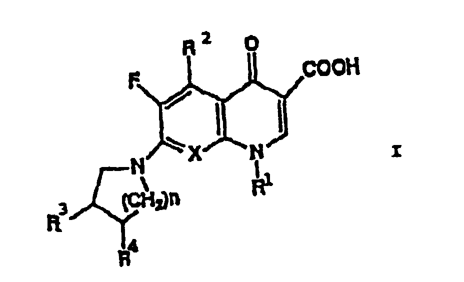 worin R' eine Cycloalkylgruppe mit 3 bis 6 Kohlenstoffatomen oder eine Phenylgruppe, die mit einer Methoxygruppe oder einer Aminogruppe oder mit ein oder zwei Halogenatomen substituiert sein kann, darstellt; R² ein Wasserstoffatom, ein Halogenatom, eine Alkylgruppe mit 1 bis 4 Kohlenstoffatomen, eine Hydroxygruppe oder eine Aminogruppe darstellt; R³ ein Wasserstoffatom, eine Hydroxygruppe oder eine Aminogruppe darstellt; R⁴ eine 1,2,3-Triazol-1-ylgruppe, eine 1,2,4-Triazol-1-ylgruppe, eine 1,2,3,4-Tetrazol-1-ylgruppe oder eine 1,2,3,4-Tetrazol-2-ylgruppe darstellt, die jeweils 1 bis 2 Substituenten, ausgewählt aus der Gruppe, bestehend aus einer Alkylgruppe mit 1 bis 4 Kohlenstoffatomen, einer COOH-Gruppe, einer CH₂NH₂-Gruppe, einer Aminogruppe und einer Phenylgruppe, tragen kann; und X N, C-H, C-F oder C-OCH₃ darstellt; n 1 ist; oder die Teilformel 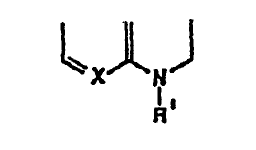 die Konstellation 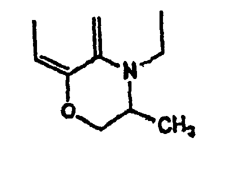 oder ein pharmazeutisch annehmbares Salz davon darstellen kann.

2. Verbindung nach Anspruch 1, worin R' eine Phenylgruppe, eine 4-Fluorphenylgruppe, eine 2,4-Difluorphenylgruppe, eine 2,4 -Dimethoxyphenylgruppe oder eine 4-Aminophenylgruppe darstellt.

3. Verbindung nach Anspruch 1, worin R' gegebenenfalls durch Chlor, Fluor, Brom, eine Methoxygruppe oder eine Aminogruppe substituiert ist.

4. Verbindung nach Anspruch 1, worin die Verbindung ein asymmetrisches Kohlenstoffatom im Pyrrolidinring aufweist, und die Verbindung das R-Isomer, das S-Isomer oder eine Mischung beider darstellt.

5. Verbindung nach Anspruch 1, worin die Verbindung zwei asymmetrische Kohlenstoffatome am Pyrrolidinring aufweist und die Verbindung ein Stereoisomer der *cis-* oder der *trans*-Konfiguration darstellt oder eine Mischung beider.

6. Verfahren zur Herstellung einer 7-substituierten 6-Fluor-1,4-dihydro-4-oxochinolin-3-carbonsäure der Formel 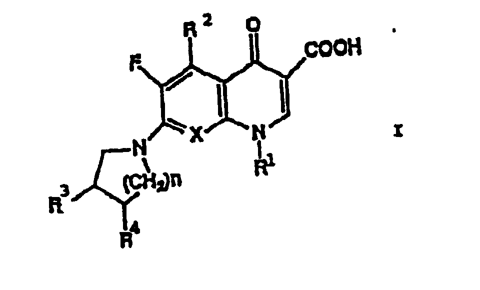 worin R' eine Cycloalkylgruppe mit 3 bis 6 Kohlenstoffatomen oder eine Phenylgruppe, die mit einer Methoxygruppe oder einer Aminogruppe oder mit ein oder zwei Halogenatomen substituiert sein kann, darstellt; R² ein Wasserstoffatom, ein Halogenatom, eine Alkylgruppe mit 1 bis 4 Kohlenstoffatomen, eine Hydroxygruppe oder eine Aminogruppe darstellt; R³ ein Wasserstoffatom, eine Hydroxygruppe oder eine Aminogruppe darstellt; R⁴ eine 1,2,3-Triazol-1-ylgruppe, eine 1,2,4-Triazol-1 -ylgruppe, eine 1,2,3,4-Tetrazol-1-ylgruppe oder eine 1,2,3,4-Tetrazol-2-ylgruppe darstellt, die jeweils 1 bis 2 Substituenten, ausgewählt aus der Gruppe, bestehend aus einer Alkylgruppe mit 1 bis 4 Kohlenstoffatomen, einer COOH-Gruppe, einer CH₂NH₂-Gruppe, einer Aminogruppe und einer Phenylgruppe, tragen kann; und X N, C-H, C-F oder C-OCH₃ darstellt; n 1 ist; oder die Teilformel 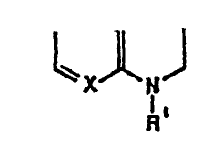 die Konstellation 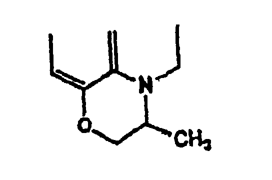 oder ein pharmazeutisch annehmbares Salz davon darstellen kann, wobei das Verfahren umfasst, dass eine Verbindung der Formel 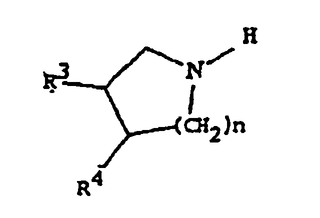 mit einer Verbindung der Formel 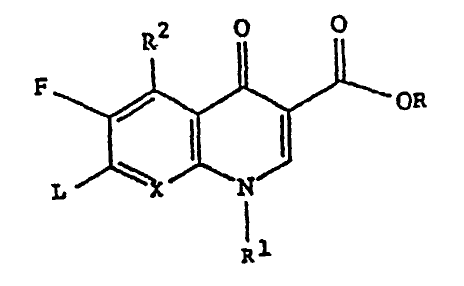 worin R¹, R², R³, R⁴, n und X die gleiche Bedeutung haben, wie sie vorstehend festgelegt wurde, L eine austretende Gruppe darstellt, die ausgewählt ist aus der Gruppe, bestehend aus Chlor, Fluor und -SO₂R₇, worin R₇ eine Alkylgruppe mit 1 bis 4 Kohlenstoffatomen oder eine substituierte oder unsubstituierte Phenylgruppe darstellt, umgesetzt wird.

7. Pharmazeutische Zusammensetzung, geeignet zur Behandlung bakterieller Infektionen, umfassend eine antibakterielle Menge einer Verbindung nach Anspruch 1 und einen pharmazeutisch annehmbaren Träger.

8. Verfahren zur Desinfizierung einer Oberfläche, die desinfiziert werden soll, das es umfasst, dass die Oberfläche mit einer Zusammensetzung behandelt wird, die eine antibakteriell wirksame Menge einer Verbindung nach Anspruch 1 enthält.

9. Verwendung einer Verbindung nach Anspruch 1 in der Herstellung eines Medikamentes zur Behandlung eines Menschen, der an einer bakteriellen Infektion leidet.

## Revendications

1. Composé acide 6-fluoro-1,4-dihydro-4-oxoquinoléine-3-carboxylique 7-substitué de formule : 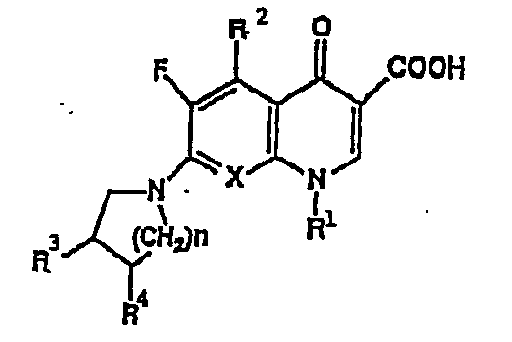 dans laquelle R¹ est un groupe cycloalkyle en C₃-C₆ ou un groupe phényle qui peut être substitué par méthoxy ou amino ou par un ou deux atomes d'halogène ; R² est un atome d'hydrogène, un atome d'halogène, un groupe alkyle en C₁-C₄, un groupe hydroxy ou un groupe amino ;
R³ est un atome d'hydrogène, hydroxy ou amino ;
R⁴ est un groupe 1,2,3-triazol-1-yle, un groupe 1,2,4-triazol-1-yle, un groupe 1,2,3,4-tétrazol-1-yle ou un groupe 1,2,3,4-tétrazol-2-yle, chacun pouvant avoir 1 à 2 substituants choisis dans le groupe constitué par les groupes alkyle en C₁-C₄, COOH, CH₂NH₂, amino et phényle ; et X est N, CH, C-F OU C-OCH₃ ; n est 1 ; ou 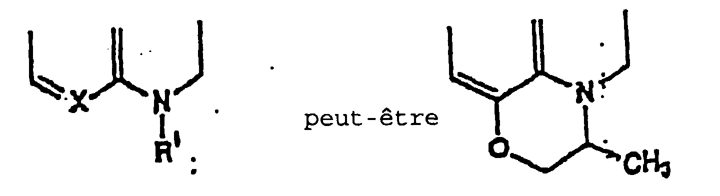 ou un sel acceptable d'un point de vue pharmaceutique de ce composé.

2. Composé selon la revendication 1, dans lequel R¹ est un groupe phényle, 4-fluorophényle, 2,4-difluorophényle, 2,4-diméthoxyphényle ou 4-aminophényle.

3. Composé selon la revendication 1, dans lequel R¹ est éventuellement substitué par chloro, fluoro, bromo, méthoxy ou amino.

4. Composé selon la revendication 1, lequel possède un atome de carbone asymétrique sur le cycle pyrrolidine, et est l'isomère R, l'isomère S, ou des mélanges de ceux-ci.

5. Composé selon la revendication 1, lequel possède deux atomes de carbone asymétrique sur le cycle pyrrolidine, et est un stéréoisomère de configuration cis, un stéréoisomère trans, ou des mélanges de ceux-ci.

6. Procédé de préparation d'un composé acide 6-fluoro-1,4-dihydro-4-oxoquinoléine-3-carboxylique 7-substitué de formule : 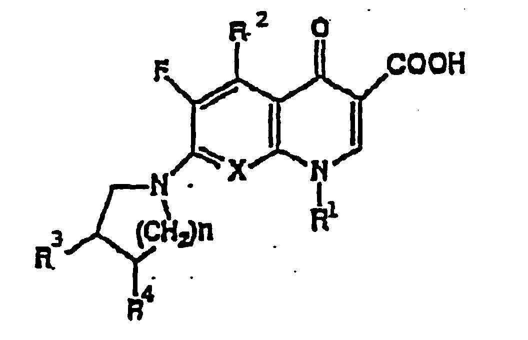 dans laquelle R¹ est un groupe cycloalkyle en C₃-C₆ ou un groupe phényle qui peut être substitué par méthoxy ou amino ou par un ou deux atomes d'halogène ; R² est un atome d'hydrogène, un atome d'halogène, un groupe alkyle en C₁-C₄, un groupe hydroxy ou un groupe amino ;
R³ est un atome d'hydrogène, hydroxy ou amino ;
R⁴ est un groupe 1,2,3-triazol-1-yle, un groupe 1,2,4-triazol-1-yle, un groupe 1,2,3,4-tétrazol-1-yle ou un groupe 1,2,3,4-tétrazol-2-yle, chacun pouvant avoir 1 à 2 substituants choisis dans le groupe constitué par les groupes alkyle en C₁-C₄, COOH, CH₂NH₂, amino et phényle ; et X est N, CH, C-F ou C-OCH₃ ; n est 1 ; ou 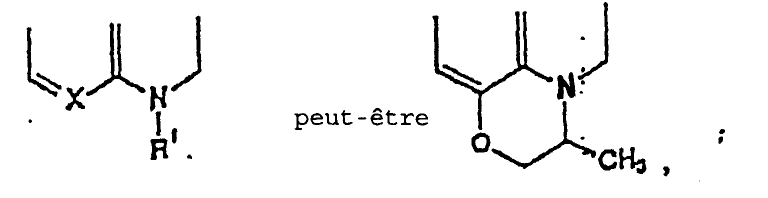 ou d'un de ses sels acceptables d'un point de vue pharmaceutique, ledit procédé comprenant la réaction d'un composé de formule 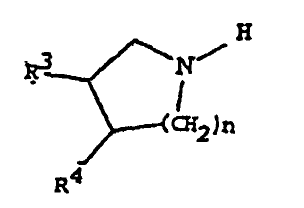 avec un composé de formule 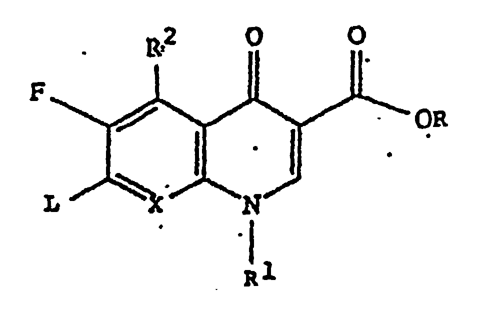 formules dans lesquelles R¹, R², R³, R⁴, n et X sont tels que définis ci-dessus ; L est un groupe partant choisi parmi le chlore, le fluor et SO₂R₇, où R₇ est un groupe alkyle en C₁-C₄ ou un groupe phényle substitué ou non substitué.

7. Composition pharmaceutique convenant au traitement d'infections bactériennes, comprenant une quantité à effet antibactérien d'un composé selon la revendication 1 et un support acceptable d'un point de vue pharmaceutique.

8. Procédé de désinfection d'une surface devant être désinfectée, qui comprend le traitement de la surface avec une composition contenant une quantité à effet antibactérien d'un composé selon la revendication 1.

9. Utilisation d'un composé selon la revendication 1, pour préparer un médicament destiné au traitement d'un patient humain souffrant d'une infection bactérienne.
